(19) Europäisches Patentamt
European Patent Office
Office européen des brevets

(11) EP 2 948 185 B1

(12) **FASCICULE DE BREVET EUROPEEN**

(45) Date de publication et mention de la délivrance du brevet:
**07.03.2018 Bulletin 2018/10**

(21) Numéro de dépôt: **14701522.6**

(22) Date de dépôt: **23.01.2014**

(51) Int Cl.:
***A61K 49/18*** *(2006.01)* ***B82Y 15/00*** *(2011.01)*

(86) Numéro de dépôt international:
**PCT/EP2014/051348**

(87) Numéro de publication internationale:
**WO 2014/114724 (31.07.2014 Gazette 2014/31)**

(54) **MAGNÉTO-ÉMULSION VECTORISÉE**

VEKTORISIERTE MAGNETISCHE EMULSION

VECTORISED MAGNETIC EMULSION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.01.2013 FR 1350584**

(43) Date de publication de la demande:
**02.12.2015 Bulletin 2015/49**

(73) Titulaire: **Guerbet**
**93420 Villepinte (FR)**

(72) Inventeurs:
- **ROBIC, Caroline**
**F-94130 Nogent Sur Marne (FR)**
- **PORT, Marc**
**F-95170 Deuil La Barre (FR)**

(74) Mandataire: **Lavoix**
**2, place d'Estienne d'Orves**
**75441 Paris Cedex 09 (FR)**

(56) Documents cités:
**US-A1- 2010 297 019**

- **JARZYNA P A ET AL: "Iron oxide core oil-in-water emulsions as a multifunctional nanoparticle platform for tumor targeting and imaging", BIOMATERIALS, ELSEVIER SCIENCE PUBLISHERS BV., BARKING, GB, vol. 30, no. 36, 1 décembre 2009 (2009-12-01), pages 6947-6954, XP026708787, ISSN: 0142-9612, DOI: 10.1016/J.BIOMATERIALS.2009.09.004 [extrait le 2009-09-23] cité dans la demande**
- **JINHO PARK ET AL: "Fibronectin extra domain B-specific aptide conjugated nanoparticles for targeted cancer imaging", JOURNAL OF CONTROLLED RELEASE, vol. 163, no. 2, 1 octobre 2012 (2012-10-01), pages 111-118, XP055083502, ISSN: 0168-3659, DOI: 10.1016/j.jconrel.2012.08.029**
- **ANGANA SENPAN ET AL: "Conquering the Dark Side: Colloidal Iron Oxide Nanoparticles", ACS NANO, vol. 3, no. 12, 22 décembre 2009 (2009-12-22), pages 3917-3926, XP055083689, ISSN: 1936-0851, DOI: 10.1021/nn900819y cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne de nouveaux systèmes optimisés de type nanoémulsions, appelés parfois magnéto-émulsions et leur utilisation comme agents de contraste, notamment en IRM.

L'administration de produits de contraste contribue à améliorer la résolution des images IRM obtenues par cette technique et la précision du diagnostic. Ainsi, l'effet de contraste peut être accentué par la présence, dans l'environnement des organes soumis à examen, de diverses espèces magnétiques, par exemple paramagnétiques, ferromagnétiques ou super-paramagnétiques.

Dans le domaine de l'imagerie diagnostique, un grand nombre de recherches ont concerné l'élaboration de nouveaux produits de contraste ciblant une région d'intérêt pour permettre d'aider au diagnostic de différentes pathologies, notamment de cancer et pour permettre aussi un suivi de l'efficacité du traitement de ces pathologies. Pour cela, l'objectif des différentes équipes de recherche, qui ont travaillé sur ces problématiques, était d'obtenir des images d'une grande qualité. Ceci n'est réalisable que par l'utilisation d'agent de contraste vectorisé, à forte sensibilité.

A ce jour, ce sont surtout des produits de contraste dans le domaine de la médecine nucléaire (par exemple, des traceurs TEP (Tomographies d'émission de positons ou PET en anglais) qui ont été, ou qui sont étudiés pour ces problématiques de suivi de traitement, en raison de l'excellente sensibilité de ces traceurs. Ces méthodes d'imagerie ont comme inconvénients que le patient est ainsi exposé à des radiations et qu'elles requièrent une logistique contraignante pour la protection du patient et la fabrication du radio-traceur (par l'intermédiaire, par exemple, d'un cyclotron).

Les substances paramagnétiques comprennent certains métaux comme le fer, le manganèse, le gadolinium à l'état ionique ou organométallique. Les substances de contraste ferromagnétiques comprennent généralement des particules d'agrégat magnétiques de taille micrométrique ou sous-micrométrique, c'est-à-dire non inférieure à 100-200 nm, par exemple des particules de ferrites, dont notamment de magnétite ($Fe_3O_4$), de maghémite ($\gamma$-$Fe_2O_3$) et autres composés minéraux magnétiques d'éléments de transition qui se comportent comme des aimants permanents. Les particules super-paramagnétiques sont habituellement de très petites particules de ferrite, dont notamment de magnétite ($Fe_3O_4$), de maghémite ($\gamma$-$Fe_2O_3$) et autres composés minéraux magnétiques d'éléments de transition, de taille inférieure à environ 100-150 nm. Contrairement aux particules ferromagnétiques, les particules superparamagnétiques ne se comportent plus comme de petits aimants autonomes du fait que leur taille est inférieure à une valeur critique, c'est-à-dire qu'elles s'alignent dans une direction préférentielle uniquement lorsqu'elles sont soumises à un champ magnétique externe. Les particules superparamagnétiques présentent avantageusement une densité d'efficacité plus élevée par rapport aux particules ferromagnétiques. Les solutions colloïdales de nanoparticules ferromagnétiques ou ferromagnétiques dans un solvant ou de l'eau sont appelées « ferrofluides».

Le manque de sensibilité de la technique d'imagerie IRM pose toutefois toujours problème. Les images obtenues avec certains produits de contraste, comme ces particules superparamagnétiques, peuvent ainsi ne pas être suffisantes pour permettre un diagnostic rapide et juste ou pour juger de l'efficacité d'un traitement d'une pathologie. Pour améliorer cette sensibilité, il faut théoriquement fournir des produits de contraste plus concentrés mais cela peut rendre ces produits moins tolérables pour le patient.

Les nanosystèmes auxquels appartiennent les nanoémulsions permettent d'accumuler des agents de contraste dans ce type de structure. En fonction du type de nanosystème utilisé, cette accumulation peut être plus ou moins importante ce qui influe directement sur la qualité des images obtenues.

Si ces produits de contraste sont des produits ciblant des récepteurs particuliers, des problèmes supplémentaires d'affinité de ces produits de contraste vis-à-vis de ces récepteurs et/ou d'accessibilité à ces récepteurs et/ou de densité de ces récepteurs peuvent aussi se poser.

Depuis quelques années sont proposées des émulsions de nanoparticules à base de fer, de type USPIO (Ultrasmall SuperParamagnetic Iron Oxide), nanoémulsions aussi parfois appelées « magnéto-émulsions », comportant, dans certains cas, des biovecteurs leur permettant de cibler des récepteurs particuliers. Ces nanoémulsions ne permettent toujours pas de résoudre de façon satisfaisante le problème de manque de sensibilité de la technique d'IRM parce que leur capacité de chargement en fer est limitée et que leur affinité vis-à-vis des récepteurs ciblés est faible.

WO 2005/014051 décrit des émulsions comprenant des nanogouttelettes, utilisables en tant qu'agent de contraste ou pour la libération d'agent thérapeutique. Ces nanogouttelettes, formées d'huile couplée à des atomes ayant un nombre Z important et en particulier supérieur à 36 (comme l'ytrium, le zirconium, l'argent ou l'or) sont recouvertes d'une couche lipidique et sont couplées à un biovecteur. US 2010/0297019 décrit des gouttelettes comprenant un coeur métallique dans une substance huileuse et une couche extérieure faite d'un composé amphiphile. La sensibilité de ce produit de contraste n'est pas suffisante dans le cas où la pathologie ciblée donne lieu à une faible expression de récepteurs.

Dans Mandal et al. (Langmuir, Vol.21, N°9, 2005) sont décrites des émulsions de nanoparticules de fer composées entre autre de maghémite ($\alpha$-$Fe_2O_3$) et d'acide oléique. Là aussi, les phases huileuses décrites (l'octane en l'occurence) dans ce document ne permettent pas d'obtenir une nanoémulsion satisfaisante.

Dans Jarzyna et al. (Biomaterials, 30, 6947-6954, 2009) sont décrites des émulsions de nanoparticules de fer

composées notamment de magnétite ($Fe_3O_4$) recouvertes d'acide oléique, la phase huileuse de ces émulsions étant principalement faite à partir d'huile de soja (en l'occurrence une huile polyinsaturée). Ces nanoémulsions sont couplées à un fluorophore Cy5.5, un agent fluorescent appartenant aux cyanines. Ce type de composé ne permet pas de vectoriser un nano-système. Cela permet juste de pouvoir constater que ce que l'on voit en imagerie est visible en fluorescence. Les nanoémusions décrites ont une capacité réduite de chargement en fer (pas plus de 15 mmoles de fer par litre d'émulsion) et ne permettent donc pas d'obtenir des images en IRM d'une qualité suffisante.

Dans Senpan et al. (JACS, Vol.3, n°12, 3917-3926, 2009) sont décrites des émulsions de nanoparticules de fer composées notamment de magnétite ($Fe_3O_4$) recouvertes d'acide oléique, la phase huileuse de ces émulsions étant principalement faite à partir d'huile d'amande (également une huile polyinsaturée). L'huile d'amande n'est de plus pas adaptée à une utilisation en injection intraveineuse et n'est a priori utilisable qu'en administration topique ou parentérale. Ces nanoémulsions sont vectorisées à l'aide de dérivés de la biotine. Outre le fait que la liaison de ce type de composé est compliquée à réaliser d'un point de vue industriel, les nanoémulsions décrites dans ce document aussi ont une capacité réduite de chargement en fer (pas plus de 80 mmoles de fer par litre d'émulsion) et ne permettent donc pas d'obtenir une sensibilité optimale en IRM.

Une dose importante de ces émulsions permettrait en théorie d'obtenir une image d'une qualité optimale, mais cela déboucherait sans aucun doute sur une saturation du récepteur ciblé si ce type d'émulsion était vectorisé. Là aussi, la sensibilité de ces produits de contraste n'est donc pas suffisante et notamment dans le cas où la pathologie ciblée donne lieu à une faible expression de récepteurs.

En voulant développer des nanoémulsions vectorisées permettant d'obtenir des images en IRM de qualité optimale, plusieurs problématiques ont été identifiées :

- De façon à obtenir des images d'une très grande qualité, il faut que les émulsions puissent accumuler en leur sein plus de 100, voire plus de 120 ou plus de 140 mmoles de fer par litre d'émulsion et il faut que ces émulsions comprenant des nanoparticules à base de fer aient une bonne affinité et une bonne sélectivité vis-à-vis du récepteur qu'elles ciblent,
- Il faut que les nanoparticules de fer aient une solubilité optimale dans la phase huileuse composant l'émulsion,
- Il faut que les huiles utilisées pour faire ces émulsions soient dans les pharmacopées, c'est-à-dire que leurs utilisations soient autorisées réglementairement pour une utilisation chez des patients,
- Il faut que ces émulsions soient stables dans le temps, c'est-à-dire qu'elles ne s'agrègent pas de façon irréversible ou qu'il n'y ait pas de destruction de l'émulsion, par exemple par coalescence ou agrégation irréversible des nanogouttelettes la constituant.

De plus, une quantité de tensioactif d'au moins environ 5% de la composition en poids, se traduit par :

- la formation dans la composition, en plus des nanogouttelettes, de micelles (dépourvues de coeur huileux) dont le retrait nécessiterait pour une production à l'échelle industrielle de centaines de tonnes de produit de contraste, des étapes de séparation et de purification complexes et coûteuses et donc une chute du rendement industriel,
- la difficulté voire l'impossibilité d'incorporer aux nanogouttelettes une quantité appropriée de ligands de ciblage biologique dont le coût est très élevé : les tensioactifs lipides amphiphiles ont un pouvoir tensioactif plus fort que les ligands de ciblage amphiphiles et vont préférentiellement former la couche autour de l'huile (et/ou la couche de lipides amphiphiles se forme à partir de ces lipides avant même que les ligands de ciblage n'aient le temps de s'intégrer au sein de cette couche).

Encore plus précisément :

- lorsque la quantité maximale de tensioactifs (tensioactif type Lipoïd® ou analogue, ligand de ciblage amphiphile, lipide pegylé le cas échéant) autour des nanogouttelettes est atteinte, les composés amphiphiles de la solution viennent former rapidement des micelles, et la solution contient alors beaucoup plus de micelles que de nanogouttelettes,
- le prix de revient industriel des nanoémulsions est pour environ au moins 80 à 90% représenté par le ligand de ciblage rendu amphiphile, on comprend donc qu'une perte de ligands de ciblage génère des surcoûts industriels beaucoup trop importants,
- si la quantité de lipides amphiphiles tensioactifs (composés non vectorisés) est trop importante, les ligands de ciblage amphiphiles ne peuvent s'intégrer de manière satisfaisante dans la couche amphiphile autour de l'huile, ce qui génère une perte d'affinité très importante et rend le produit inapproprié pour un ciblage spécifique du territoire pathologique.

Les nanoémulsions selon l'invention sont biovectorisées (par la présence du ligand de cliblage) car destinées

à l'imagerie moléculaire diagnostique. Dans l'art antérieur, sont décrites plusieurs façons de vectoriser des émulsions. Les deux modes principaux de vectorisation sont le greffage de ligands de ciblage sur un ou plusieurs composés de l'émulsion après synthèse de l'émulsion ou le greffage de ligands de ciblage sur un ou plusieurs composés de l'émulsion puis synthèse de l'émulsion comprenant ce composé et les autres constituants (on parlera alors d'incorporation du ligand de ciblage).

Dans le cas où est fait le choix du greffage de ligands de ciblage sur des composés de l'émulsion après synthèse de l'émulsion, des impuretés se forment lors du greffage. Cela pose un problème important puisque ces impuretés sont sur l'émulsion, il est difficile, voire impossible, de les éliminer.

Dans le cas où est fait le choix de l'incorporation de ligands de ciblage, les nanogouttelettes de la nanoémulsion présentent, incorporés au niveau de la couche formée par les tensioactifs, un ou plusieurs ligands de ciblage spécifiques qui viennent reconnaître spécifiquement par interaction moléculaire (affinité cible/ligand) la cible biologique (récepteur, enzyme ...) dont l'expression est modifiée dans la zone pathologique. Ces ligands de ciblage sont également désignés « biovecteurs » ou « ligands de reconnaissance » par l'homme du métier.

Or un problème technique très difficile à résoudre est précisément d'incorporer de manière appropriée et stable dans la durée un ou plusieurs ligands de ciblage pour l'imagerie moléculaire, en quantité suffisante pour obtenir une spécificité du marquage, mais pas trop élevée pour éviter des prix de revient industriels trop élevés.

Au vu de cet art antérieur complexe, on voit la difficulté d'obtenir des nanoémulsions vectorisées pour l'IRM, à la fois chimiquement industrialisables et stables, biologiquement performantes et permettant d'obtenir des images en IRM d'une grande qualité.

Des nanoémulsions comprenant des particules superparamagnétiques, encapsulées dans des nanogouttelettes vectorisées et résolvant tous les problèmes techniques de l'art antérieur ont été obtenues.

Dans la suite du texte, les termes « nanogouttelette », « gouttelette de nanoémulsion » ou « nano-objet » seront équivalents.

En particulier, des compositions optimisées ont été sélectionnées avec des capacités améliorées de chargement en fer et comprenant suffisamment de tensioactif pour stabiliser la taille des nanogouttelettes, mais pas trop pour éviter l'incorporation insuffisante des ligands de ciblage.

Dans les nanoémulsions selon l'invention, le ligand de ciblage doit pouvoir se loger au sein de l'interface huile/eau, en s'ancrant dans la membrane/pellicule amphiphile des tensioactifs. Il n'était pas du tout évident pour l'homme du métier de trouver les bons composés et les bons rapports de quantités entre les tensioactifs, l'huile, et les ligands de ciblage, qui permettent d'obtenir des nanoémulsions avec une efficacité améliorée en imagerie moléculaire et sans une perte de ligands de ciblage très onéreux.

A cet effet, l'invention concerne, selon un premier aspect, une composition de nanoémulsion huile dans eau, notamment pour IRM, comprenant :

- de 50 à 90% en poids, avantageusement de 60 à 85% en poids, plus avantageusement de 75 à 85% en poids, encore plus avantageusement de 78 à 82% en poids de phase aqueuse,
- de 9,5 à 49,5% en poids, avantageusement de 9,5 à 39,5% en poids, plus avantageusement de 14,5 à 24,5% en poids, encore plus avantageusement de 17,5 à 21,5% en poids de phase lipidique sous forme de nanogouttelettes comprenant :
  - une huile, et
  - des particules magnétiques (p) à base d'un composé du fer, lesdites particules magnétiques (p) étant recouvertes par un ou plusieurs acides gras en C8-C22, avantageusement en C14-C18, plus avantageusement en C16-C18, encore plus avantageusement en C18,
- de 0,38 à 4,95% en poids, avantageusement de 0,5 à 2 % en poids d'un mélange de tensioactifs à l'interface entre les phases aqueuse et lipidique, le mélange de tensioactifs :
  - comprenant au moins un lipide amphiphile et au moins un ligand de ciblage amphiphile, et
  - représentant de 4 à 10% en poids, avantageusement de 5 à 8 % en poids par rapport à l'huile de la phase lipidique;

caractérisée en ce que l'huile de la phase lipidique comprend au moins 70%, avantageusement au moins 80%, de façon avantageuse au moins 95% en poids, notamment au moins 97 %, de glycérides d'acides gras saturés en C6-C18, avantageusement en C6-C14, plus avantageusement en C6-C10 et en ce que la composition comprend plus de 100 mmoles, avantageusement plus de 120 mmoles, encore plus avantageusement plus de 140 mmoles de Fer par litre de composition.

De façon avantageuse, dans la composition selon l'invention, le total des pourcentages de phase aqueuse, de

phase lipidique et de tensioactif à l'interface de ces deux phases est égal à 100%.
On entend par le terme « acide gras » désigner des acides carboxyliques aliphatiques présentant une chaîne carbonée d'au moins 4 atomes de carbone. Les acides gras naturels possèdent une chaîne carbonée de 4 à 28 atomes de carbone (généralement un nombre pair). On parle d' « acide gras à longue chaîne » pour une longueur de 14 à 22 carbones et à très longue chaîne s'il y a plus de 22 carbones. On parle au contraire d' « acide gras à courte chaîne » pour une longueur de 6 à 10 carbones, en particulier 8 ou 10 atomes de carbone. L'homme du métier connaît la nomenclature associée et en particulier utilise :

- Ci-Cp pour désigner une fourchette d'acides gras en Ci à Cp
- Ci+Cp, le total des acides gras en Ci et des acides gras en Cp

Par exemple :

- les acides gras de 14 à 18 atomes de carbone s'écrivent « acides gras en C14-C18 »
- le total des acides gras en C16 et des acides gras en C18 s'écrit C16 +C18.
- pour un acide gras insaturé, un homme du métier utilisera la nomenclature suivante Ci : x n-N où N sera la position de la double liaison dans l'acide gras insaturé en partant du carbone opposé au groupe acide, i est le nombre d'atomes de carbone de l'acide gras, x est le nombre de double liaisons (insaturations) de cet acide gras. L'acide oléique, sera par exemple désigné par la nomenclature (C18 :1 n-9).

La concentration en fer de la composition de nanoémulsion est mesurée par les techniques connues de l'homme du métier et par exemple par spectroscopie d'émission atomique. De façon préférentielle, la composition de nanoémulsion selon l'invention comprend de 100 à 300 mmoles de Fer par litre de composition, plus préférentiellement de 120 à 200 mmoles de Fer par litre de composition, encore plus préférentiellement de 140 à 160 mmoles de Fer par litre de composition.
Des précisions sur les différents constituants de la composition sont données ci-après.

**Phase aqueuse**

La phase aqueuse est avantageusement de l'eau ou une solution aqueuse pharmaceutiquement acceptable telle qu'une solution saline ou une solution tampon. Elle pourra notamment comprendre certains additifs tels que du glycérol ou du mannitol.

**Huile de la phase lipidique**

Très avantageusement la phase lipidique est constituée par l'huile et par les particules magnétiques (p).
Les glycérides d'acides gras saturés de l'huile de la phase lipidique se trouvent avantageusement sous la forme de triglycérides d'acides gras saturés. L'huile comprend au moins 70%, de préférence au moins 80, 90, 95, 97 % en poids de glycérides d'acides gras saturés en C6-C10. Cette huile aura comme avantages d'être bien adaptée pour des formulations pharmaceutiques injectables d'agents de contraste, de ne pas être sensible à l'oxydation (permettant ainsi une conservation de plusieurs mois du produit la comprenant et aucune altération du comportement paramagnétique du produit pour les examens d'imagerie médicale) et surtout de permettre à la composition de nanoémulsion dont la phase lipidique comprend cette huile ou au produit de contraste comprenant cette composition d'avoir une très bonne affinité vis-à-vis du récepteur qu'elle cible.
Très avantageusement, l'huile comprend moins de 10%, de préférence moins de 5% de glycérides d'acides gras insaturés, en particulier moins de 5%, et de préférence moins de 2%, moins de 1% en poids de glycérides d'acides gras insaturés en C14-C18 ou en C14-C22.
L'huile de la phase lipidique comprend préférentiellement ou peut être constituée d'un mélange de diglycérides et/ou de triglycérides d'un ou plusieurs acides gras choisis parmi l'acide caprylique, l'acide caprique, l'acide linoléique et l'acide succinique ou l'un de leurs dérivés.
Par dérivés d'acide caprylique, d'acide caprique, d'acide linoléique, d'acide succinique, on entend des dérivés méthylés, hydroperoxylés, hydroxylés, oxoylés, époxylés, méthoxylés, halogénés, aminés, cyanylés, nitrosylés ou thiolés de ces différents acides gras.
De façon avantageuse, l'huile de la phase lipidique comprend un mélange de triglycérides d'acide caprylique et d'acide caprique.
De façon avantageuse, l'huile de la phase lipidique comprend plus de 80, 85, 90, 95% en poids d'un mélange de triglycérides d'acide caprylique et d'acide caprique.
Par exemple, l'huile de la phase lipidique est l'huile de coco ou l'huile MIGLYOL®, notamment de formule :

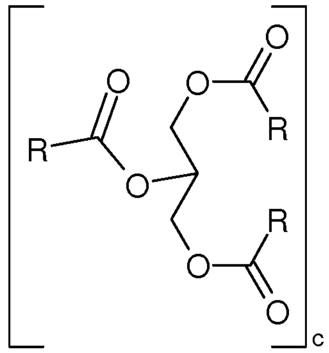

(R = C8 + C10) > 95 %

ou l'un des ses dérivés connus, par exemple l'huile MIGLYOL® 810, l'huile MIGLYOL® 812 (caprylic/capric triglyceride), l'huile MIGLYOL® 818 (caprylic/capric/linoleic triglyceride), l'huile MIGLYOL® 612 (glyceryl trihexanoate) ou d'autres dérivés MIGLYOL® propylène glycol dicaprylate dicaprate.

L'huile de coco a la composition suivante :

| Nature de l'acide gras des glycérides d'acide gras | Concentration (en % m/m) |
|---|---|
| Acide caprylique (C8:0) | 6 - 9% |
| Acide caprique (C10:0) | 6-10% |
| Acide laurique (C12:0) | 44 - 51% |
| Acide myristique (C14:0) | 13 - 18% |
| Acide palmitique (C16:0) | 8 - 10 % |
| Acide stéarique (C18:0) | 1 - 3 % |
| Acide oléique (C18:1 n-9) | 0,5 - 7,5 % |
| Acide linoléique (C18:2 n-6) | <2.5% |

L'huile de la phase lipidique est préférentiellement une huile MIGLYOL®.
Par exemple l'huile Miglyol® 812 a la composition suivante :

| Acide gras des glycérides d'acide gras | Concentration (m/m) |
|---|---|
| Acide caproïque (C6 : 0) | max 2 % |
| Acide caprylique (C8 : 0) | 50 à 65 % |
| Acide caprique (C10 : 0) | 30 à 45 % |
| Acide laurique (C12 : 0) | max 2 % |
| Acide myristique (C14 : 0) | max 1 % |
| Acide linoléique (C18 : 2 n-6) | - |

L'huile Miglyol® 818 a elle la composition suivante :

| Acide gras des glycérides d'acide gras | Concentration (m/m) |
|---|---|
| Acide caproïque (C6 : 0) | max 2 % |
| Acide caprylique (C8 : 0) | 45 à 65 % |
| Acide caprique (C10 : 0) | 30 à 45 % |
| Acide laurique (C12 : 0) | max 3 % |

(suite)

| Acide gras des glycérides d'acide gras | Concentration (m/m) |
|---|---|
| Acide myristique (C14 : 0) | max 1 % |
| Acide linoléique (C18 : 2 n-6) | 2 à 5 % |

Selon des variantes, l'huile de la phase lipidique est un mélange de glycérides d'acides gras saturés comprenant au moins 70%, de préférence au moins 80, 90, 95% en poids de glycérides d'acides gras saturés de 6 à 10 atomes de carbone.

De préférence, les glycérides d'acides gras saturés de l'huile de la phase lipidique sont sous forme de mono, di ou triglycérides, de préférence sous forme de triglycérides.

De manière préférée, l'huile de la phase lipidique des émulsions comprend des glycérides d'acides gras saturés dont les acides gras saturés sont dans les variantes suivantes :

- C6-C18 > 70% en poids, préférentiellement C6-C18 > 80% en poids, plus préférentiellement C6-C18 > 95% en poids, et encore plus préférentiellement C6-C18 > 98% en poids, ou
- C6-C14 > 70% en poids, préférentiellement C6-C14 > 80% en poids, plus préférentiellement C6-C14 >95% en poids, et encore plus préférentiellement C6-C14 > 98% en poids ou
- C8+C10 > 70% en poids, préférentiellement C8+C10 > 80% en poids, plus préférentiellement C8+C10 >95% en poids, et encore plus préférentiellement C8+C10 > 98% en poids ou
- C8 de 40 à 70% en poids, préférentiellement de 50 à 65% en poids et/ou C10 de 20 à 50% en poids, plus préférentiellement de 30 à 45% en poids, le total C8+C10 étant supérieur à 80% en poids.

Il a été constaté qu'au-delà de 49,5% en poids de phase lipidique dans la composition, celle-ci adopte un comportement trop rhéofluidifiant et/ou une viscosité (la viscosité devenant alors supérieure à des valeurs de 4 à 5 mPa.s) non adaptées pour l'injection intraveineuse.

Il est précisé que compte tenu notamment du volume injectable aux patients, de l'ordre de 10 à 50 ml, l'huile est utilisée à un taux suffisamment élevé, d'au moins 9,5% en poids par rapport au poids de la composition, afin d'avoir une solution suffisamment concentrée à la fois en gouttelettes et en fer pour obtenir un signal en IRM suffisant. Il est nécessaire d'avoir une concentration adaptée pour la durée d'injection, le moment de l'acquisition du signal et le traitement associé des données par le praticien. Une solution trop diluée la rendrait inutilisable pour les examens d'imagerie médicale.

**Mélange de tensioactifs à l'interface entre les phases aqueuse et lipidique**

On rappelle que le terme « tensioactif » ou « surfactant » fait référence à un composé à structure amphiphile qui lui confère une affinité particulière pour les interfaces de type huile/eau ou eau/huile ce qui lui donne la capacité d'abaisser l'énergie libre de ces interfaces et de stabiliser des systèmes dispersés.

L'homme du métier comprend que le mélange de tensioactifs à l'interface est représenté par l'ensemble des tensioactifs utilisés, c'est-à-dire, comme expliqué en détail dans la demande : des lipides amphiphiles, des ligands de ciblage amphiphiles, et le cas échéant d'autres composés tels que des lipides pégylés (lipides couplés à des PEG). De par leur structure amphiphile, les ligands de ciblage amphiphiles jouent un rôle de tensioactif.

Des exemples de ces composés seront donnés dans les pages suivantes.

Les nanogouttelettes comprennent chacune un nombre de ligand de ciblage amphiphile de l'ordre de 100 à 5000, notamment 500 à 4000, notamment 1800 à 3500 (par exemple 2000) ce qui permet le ciblage efficace selon l'affinité et la multivalence du ligand de ciblage. Les résultats biologiques obtenus grâce aux nanoémulsions selon l'invention montrent de plus que les ligands de ciblage sont avantageusement répartis sur l'ensemble de la surface externe des nanogouttelettes, ce qui se traduit par une multivalence optimisée des ligands de ciblage.

Les ligands de ciblage amphiphiles représentent avantageusement de 0,01 à 10% mole/mole de la quantité totale de tensioactifs du mélange de tensioactifs, plus avantageusement de 0,05 à 5%, notamment de 0,05 à 3 %.

Par un pourcentage (%) « mole/mole» de la quantité totale de tensioactif, on entend le nombre de moles de ligand de ciblage amphiphile pour 100 moles de tensioactifs totaux du mélange de tensioactifs.

Le produit de contraste injecté comprenant les compositions de nanoémulsions décrites a une affinité avantageusement de l'ordre de 1 pM à 100 nM, notamment 1 pM à 50 nM, avantageusement 1 pM à 100 pM (l'affinité par ligand de ciblage amphiphile, autour de 1 nM à 1 $\mu$M est divisée par le nombre de ligands de ciblage par nanogouttelette).

De manière préférée, le mélange de tensioactifs à l'interface entre les phases aqueuse et lipidique de la

nanoémulsion selon l'invention, comprend de 80 à 96,95 % mole/mole de lipide amphiphile, de 3 à 15% mole/mole de lipide pégylé et de 0,05 à 5% mole/mole d'un ligand de ciblage amphiphile.

**Particules métalliques (p)**

Par "particules à base d'un composé du fer", on entend, au sens de la présente description, des particules comprenant ou constituées d'un composé du fer, comprenant généralement du fer (III), généralement un oxyde ou un hydroxyde de fer.

En règle générale, les particules magnétiques (p) sont composées en tout ou partie d'hydroxyde de fer ; d'oxyde de fer hydraté ; de ferrites ; d'oxydes de fer mixtes tels que des oxydes de fer mixtes de cobalt, de nickel, de manganèse, de béryllium, de magnésium, de calcium, de baryum, de strontium, de cuivre, de zinc ou de platine ; ou d'un mélange de ceux-ci.

Au sens de la présente demande, le terme de "ferrite" désigne les oxydes de fer de formule générale [x $Fe_2O_3$, y $MO_z$], où M désigne un métal magnétisable sous l'effet d'un champ magnétique tel que Fe, Co, Ru, Mg, Mn, le métal magnétisable pouvant être éventuellement radioactif.

De façon préférentielle, les particules magnétiques (p) des compositions de l'invention comprennent une ferrite, notamment la maghémite (γ $Fe_2O_3$) ou la magnétite ($Fe_3O_4$), ou encore les ferrites mixtes de cobalt ($Fe_2CoO_4$) ou de manganèse ($Fe_2MnO_4$). Dans ce cadre, on préfère tout particulièrement les particules magnétiques (p) composées en tout ou partie d'une ferrite, et de préférence essentiellement (c'est-à-dire plus de 90% préférentiellement plus de 95%, encore plus préférentiellement plus de 98% en poids), de maghémite ou de magnétite ou d'un mélange de ceux-ci.

Les particules magnétiques (p) sont préférentiellement des particules magnétiques acides.

Les particules magnétiques (p) des compositions selon l'invention présentent en surface des sites hydroxyles protonés en milieu acide (c'est-à-dire un milieu ayant un pH de 1 à 3,5, préférentiellement de 2 à 3) qui correspondent plus précisément à l'espèce $Fe\text{-}OH_2^+$ résultant d'une interaction très forte entre un ion $Fe^{3+}$ à la surface de la particule et une molécule d'eau acide ($H_3O^+$). Ces protons peuvent être considérés comme constitutifs de la particule d'après les travaux de J. Lyklema et al., Materials Science Research, 1984, 17, p. 1-24.

Selon une variante particulièrement préférée, les particules magnétiques (p) sont superparamagnétiques.

Les particules magnétiques (p) avant d'être recouvertes par un ou plusieurs acides gras possèdent alors, de préférence, un diamètre hydrodynamique de 5 à 200 nm, mieux encore de 5 à 60 nm ou de 5 à 20 nm.

De façon très avantageuse, les particules magnétiques (p) à base d'un composé du fer, sont recouvertes par un acide gras insaturé, préférentiellement mono-insaturé, encore plus préférentiellement par de l'acide oléique (C 18 :1 n-9).

Cet acide gras a comme avantage de rendre optimale la solubilisation des particules magnétiques dans les huiles de la phase lipidique selon l'invention.

**Autres caractéristiques des nanoémulsions**

Les gouttelettes des nanoémulsions selon l'invention ont une taille suffisamment petite pour leur permettre de circuler dans les milieux biologiques sans dégradation du produit, jusqu'à la cible du ligand de ciblage amphiphile inséré dans les nanogouttelettes grâce à son groupe lipophile. La taille des nanogouttelettes est typiquement de 30 à 300 nm, avantageusement 50 à 250 nm, notamment 100 à 220 nm, en particulier 180 à 210 nm. La taille des nanoémulsions est mesurée en utilisant la méthode PCS (voir détails ci-après).

De plus, les formulations obtenues sont iso-osmolaires (c'est-à-dire que leur osmolarité est identique à celle du plasma), ce qui évite des désagréments pour le patient lors de l'injection. En outre, la quantité de ligands de ciblage greffés aux nanogouttelettes est très bien adaptée pour l'imagerie IRM. La composition est de plus capable de supporter la stérilisation à la chaleur, typiquement par autoclavage.

**Modes de réalisation préférés**

Selon des modes de réalisation préférés, la composition de nanoémulsion selon l'invention a pour composition en poids :

1) de 50 à 90% en poids, avantageusement de 60 à 85% en poids, plus avantageusement de 75 à 85% en poids, encore plus avantageusement de 78 à 82% en poids de phase aqueuse,
2) de 9,5 à 49,5% en poids, avantageusement de 9,5 à 39,5% en poids, plus avantageusement de 14,5 à 24,5% en poids, encore plus avantageusement de 17,5 à 21,5 % en poids de phase lipidique,
3) de 0,38 à 4,95% en poids de mélange de tensioactifs (de préférence de 4 à 10% de la phase lipidique), préférentiellement de 0,5 à 2% en poids de mélange de tensioactifs, le mélange de tensioactifs comprenant de 90 à

99,99%, avantageusement de 95 à 99,95%, plus avantageusement de 97 à 99,95% en mole/mole de lipide amphiphile, et de 0,01 à 10% mole/mole, avantageusement 0,05 à 5 %, plus avantageusement 0,05 à 3 % de ligand de ciblage amphiphile, étant précisé que le total des pourcentages de 1), 2) et 3) est égal à 100%.

Selon des modes de réalisation préférés, la nanoémulsion a pour composition en poids :

1) de 50 à 90% en poids, avantageusement 60 à 85% en poids, plus avantageusement 75 à 85% en poids, encore plus avantageusement 78 à 82% en poids de phase aqueuse,
2) de 9,5 à 49,5% en poids, avantageusement de 9,5 à 39,5% en poids, plus avantageusement de 14,5 à 24,5% en poids, encore plus avantageusement de 17,5 à 21,5 % en poids de phase lipidique,
3) de 0,38 à 4.95% en poids de mélange de tensioactifs, le mélange de tensioactifs comprenant de 95 à 99,95% mole/mole de lipide amphiphile et 0,05 à 5% mole/mole, notamment 0,05 à 3 % mole/mole de ligand de ciblage amphiphile, étant précisé que le total des pourcentages de 1), 2) et 3) est égal à 100%.

Selon des réalisations préférées, la nanoémulsion a pour composition en poids :

1) de 50 à 90%, de préférence 60 à 85%, avantageusement 75 à 85%, plus avantageusement 78 à 82%, notamment 79 à 81 % en poids de phase aqueuse,
2) de 9,5 à 49,5%, de préférence 9,5 à 39,5%, avantageusement 14,5 à 24,5%, plus avantageusement 17.5 à 21.5% en poids de phase lipidique comprenant une huile, l'huile comprenant au moins 70%, de préférence au moins 80, 90, 95% en poids de glycérides d'acides gras saturés C6-C14, de préférence C6-C10,
3) de 0,38 à 4,95%, avantageusement 0,38 à 3,95%, plus avantageusement 0,5 à 1,5% en poids de mélange de tensioactifs,

étant précisé que le total des pourcentages de 1), 2) et 3) est égal à 100%.

Selon des réalisations préférées, le mélange des tensioactifs comprend (en % mole/mole de la quantité totale de tensioactifs) :

3.1) de 50 à 99,99%, avantageusement de 65 à 97,95 %, plus avantageusement 77 à 97,95%, encore plus avantageusement de 80 à 96,95% de lipide amphiphile,
3.2) de 0,01 à 10%, avantageusement de 0,05 à 5%, encore plus avantageusement de 0,05 à 3% de ligand de ciblage amphiphile,
3.3) de 0 à 40%, avantageusement de 2 à 30%, plus avantageusement de 2 à 20% , encore plus avantageusement de 3 à 15% de lipide pégylé, étant précisé que le total des pourcentages de 3.1), 3.2) et 3.3) est égal à 100%.

En particulier, les modes de réalisations suivants sont avantageuses:

| % en poids de phase aqueuse par rapport à la composition (1) | % en poids de phase lipidique par rapport à la composition (2) | % en poids de mélange de tensioactifs par rapport à la phase lipidique (3) | % en poids de mélange de tensioactifs par rapport à la composition (4) **(*)** | Concentration en fer (mmoles/litre de composition) |
|---|---|---|---|---|
| 50 - 90 | 9,5 - 40 | 4 - 10 de (2) | [0,38 - 4] % | 100 - 300 |
| 70 - 90 | 9,5 - 29.5 | 4 - 10 de (2) | [0,38 - 2,95] % | 100 - 300 |
| 75 - 85 | 14 - 25 | 4 - 10 de (2) | [0,56 - 2,5] % | 100 - 300 |
| 78 - 82 | 17 - 21 | 4 - 10 de (2) | [0,68 - 2,1] % | 100 - 300 |
| 75 - 85 | 14 - 25 | 5 - 8 de (2) | [0,7 - 2] % | 100 - 300 |

(suite)

| % en poids de phase aqueuse par rapport à la composition (1) | % en poids de phase lipidique par rapport à la composition (2) | % en poids de mélange de tensioactifs par rapport à la phase lipidique (3) | % en poids de mélange de tensioactifs par rapport à la composition (4) **(*)** | Concentration en fer (mmoles/litre de composition) |
|---|---|---|---|---|
| 78 - 82 | 17 - 21 | 5 - 8 de (2) | [0,85 - 1,68] % | 100 - 300 |
| Etant précisé que le total (1) + (2) + (4) = 100%<br>(*) les gammes de la colonne 4 correspondent aux pourcentages de la colonne 3 multipliés par les pourcentages de la colonne 2 (les plus faibles avec les plus faibles et les plus forts avec les plus forts). Par exemple, la gamme [0,38 - 4] correspond à 4% (pourcentage précisé en colonne 3) x 9,5 (pourcentage précisé en colonne 2) = 0,38 % et 10% (pourcentage précisé en colonne 3) x 40 (pourcentage précisé en colonne 2) = 4 %. Ces gammes sont préférées notamment dans la mesure où elles permettent d'obtenir une taille de nanogouttelettes d'émulsion de 150 à 300 nm, et en particulier autour de 180 à 210 nm. | | | | |

La taille et la stabilité des nanogouttelettes d'émulsion sont très satisfaisantes, ainsi que la viscosité (de l'ordre de 1 à 3 mPa.s). Leur comportement est newtonien, ce qui constitue un avantage important pour des solutions pharmaceutiques injectables.
On a par exemple les gammes de proportions suivantes des constituants.

| % en poids de phase aqueuse (1) | % en poids de phase lipidique (2) | % en poids de mélange de tensioactifs par rapport à la phase lipidique (3) | Teneur % (mole/mole) en lipide amphiphile dans le mélange de tensioactifs | Teneur % (mole/mole) en lipide pégylé dans le mélange de tensioactif | Teneur % (mole/mole) en ligand de ciblage amphiphile dans le mélange de tensioactifs | Concentration en fer (mmoles/litre de composition) |
|---|---|---|---|---|---|---|
| 75 à 85, de préférence 78 à 82, de préférence 78,6 | 14 à 25, de préférence 17 à 21, de préférence 20 | 5 à 10, de préférence 5 à 8, de préférence 7,2 | 95 à 99,95 | 0 | 0,05 à 5, de préférence 0,05 à 3 | 100 à 300, de préférence 120 à 200, de préférence 140 à 160 |
| 75 à 85, de préférence 78 à 82, de préférence 78,6 | 14 à 25, de préférence 17 à 21, de préférence 20 | 5 à 10, de préférence 5 à 8, de préférence 7,2 | 80 à 96,95 | 3 à 15, de préférence 5 à 15 | 0,05 à 5, de préférence 0,05 à 3 | 100 à 300, de préférence 120 à 200, de préférence 140 à 160 |
| 75 à 85, de préférence 78 à 82, de préférence 78,6 | 14 à 25, de préférence 17 à 21, de préférence 20 | 5 à 10, de préférence 5 à 8, de préférence 7,2 | 90 à 99,95 | 0 à 5 | 0,05 à 5, de préférence 0,05 à 3 | 100 à 300, de préférence 120 à 200, de préférence 140 à 160 |

le total dans le mélange de tensioactits des teneurs en lipides amphiphiles, lipides pégylés, ligands de ciblage amphiphiles, étant de 100%.

**Lipides amphiphiles**

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone.
Ils peuvent être choisis parmi les phospholipides, les cholestérols, les lysolipides, les sphingomyélines, les

tocophérols, les glycolipides, les stéarylamines, les cardiolipines d'origine naturelle ou synthétique ; les molécules composées d'un acide gras couplé à un groupement lipophile par une fonction éther ou ester, tels que les esters de sorbitan comme par exemple les monooléate et monolaurate de sorbitan ; les lipides polymérisés ; les esters de sucre tels que les mono-et di-laurate, mono- et di-palmitate, mono- et distéarate de saccharose; lesdits lipides amphiphiles pouvant être utilisés seuls ou en mélanges.

Avantageusement, le lipide amphiphile est un phospholipide, de préférence choisi parmi : phosphatidylcholine (appelée aussi lécithine), dioléoylphosphatidylcholine, dimyristoylphosphatidylcholine, dipalmitoylphosphatidylcholine, distéaroylphosphatidylcholine, phosphatidyléthanolamine, sphingomyéline, phosphatidylsérine, phosphatidylinositol. La lécithine est un lipide amphiphile préféré.

Avantageusement le lipide amphiphile est un lipoïde, notamment l'EPC (Egg Phosphatidyl Choline et ses dérivés connus notamment d'Avanti Polar Lipids) ou le lipoid® S75 de formule :

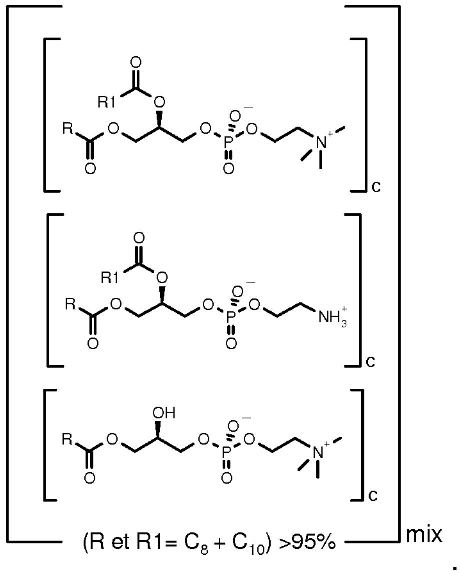

|  | **Proportions (m/m)** |
|---|---|
| Phosphatidylcholine (+LPC) | 68 à 73 % |
| Phosphatidylethanolamine | 7 à 10 % |
| Lysophosphatidylcholine | < à 3 % |
| Phosphore | 3,4 à 3,7 % |

Selon un mode de réalisation particulier, tout ou partie du lipide amphiphile peut posséder une fonction réactive, telle qu'un groupe maléimide, thiol, amine, ester, oxyamine ou aldéhyde. La présence de fonctions réactives permet le greffage de composés fonctionnels au niveau de l'interface.

**Lipides pégylés**

On pourra utiliser pour la phase lipidique, en plus du lipide amphiphile et du ligand de ciblage amphiphile, de manière non obligatoire, et en particulier afin d'agir sur le caractère furtif du produit dans l'organisme (c'est-à-dire en permettant de retarder l'élimination du produit par le système réticulo-endothélial), des lipides pégylés c'est-à-dire porteurs de groupes oxyde de polyéthylène (PEG), tels que le polyéthylèneglycol/phosphatidyléthanolamine (PEG-PE). Par « polyéthylèneglycol» PEG, au sens de la présente demande, on désigne de façon générale, des composés comprenant une chaîne $-CH_2-(CH_2-O-CH_2)_k-CH_2OR_3$ dans laquelle k est un nombre entier variant de 2 à 100 (par exemple 2, 4, 6, 10, 50), et $R_3$ est choisi parmi H, alkyle ou -(CO)Alk, le terme "alkyle" ou "Alk" désignant ici un groupe aliphatique hydrocarboné, linéaire ou ramifié, ayant environ de 1 à 6 atomes de carbone dans la chaîne. Le terme « polyéthylèneglycol » tel qu'employé ici englobe notamment les composés aminopolyéthylèneglycols. Comme exemple de lipide pégylé, on citera notamment les PEG 350, 750, 2000, 3000, 5000, modifiés par ajout de groupements amphiphiles pour s'insérer au sein de la couche de tensioactifs de la nanogouttelettes, notamment :

- 1,2-Distearoyl-sn-Glycero-3-Phosphoethanolamine-N-[Methoxy(Polyethylene glycol)-350]
- 1,2-Distearoyl-sn-Glycero-3-Phosphoethanolamine-N-[Methoxy(Polyethylene glycol)-550],
- 1,2-Distearoyl-sn-Glycero-3-Phosphoethanolamine-N-[Methoxy(Polyethylene glycol)-750].

On utilisera notamment le lipide pégylé :

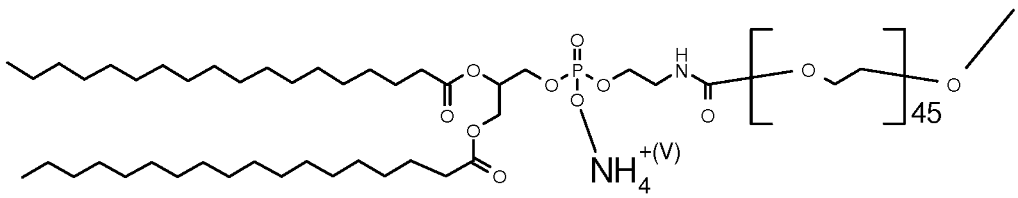

**Ligands de ciblage amphiphiles**

Les nanoémulsions sont vectorisées à l'aide de ligands de ciblage amphiphiles. La nanoémulsion comprend au moins un ligand de ciblage d'une zone pathologique ancré à la nanogouttelette, typiquement grâce au groupe lipophile du ligand de ciblage amphiphile.
Les nanoémulsions ont en outre l'avantage de pouvoir contrôler le type et la quantité de ligands de ciblage amphiphiles, et notamment de pouvoir incorporer des ligands de ciblage amphiphiles différents. Par exemple, une nanogouttelette comprendra :

- un ligand de ciblage amphiphile qui permet l'accession à une zone physiologique pathologique, par exemple comprenant un ligand de ciblage de passage à travers la BHE (barrière hémato encéphalique)
- un autre ligand de ciblage amphiphile qui permet le ciblage d'un marqueur biologique cible surexprimé par certaines cellules de cette zone pathologique.

Les interactions moléculaires entre le ligand de ciblage du ligand de ciblage amphiphile et le marqueur biologique cible permettent la captation des nanogouttelettes au niveau de la zone pathologique, et l'imagerie IRM qui en résulte permet de localiser précisément la zone pathologique.
Avantageusement, le nombre de ligands de ciblage amphiphiles par gouttelette de nanoémulsion est d'au moins 50 et typiquement de l'ordre de 500, 1000, 2000, 3000, 5000, 10 000.
Le dosage du ligand de ciblage amphiphile peut par exemple être effectué par spectrométrie de masse MALDI-TOF. Le nombre de ligands de ciblage amphiphiles par gouttelette de nanoémulsion est calculé à partir des résultats de mesure de diamètre hydrodynamique (Z ave) obtenus par PCS, du volume d'huile dans l'émulsion et de la concentration en ligands de ciblage amphiphiles obtenue par la méthode ci-dessus.
A titre de ligands de ciblage préférés du ligand de ciblage amphiphile, on citera des ligands de cibles biologiques dont l'expression est modifiée dans une zone pathologique (une tumeur par exemple), par rapport à la zone saine. De façon très préférentielle, on peut citer, comme cible biologique entrant dans cette définition, les intégrines et notamment l'intégrine $\alpha V\beta 3$, qui est le récepteur de la vitronectine et qui est en fait composé de deux parties : l'intégrine alpha V et l'intégrine beta 3 (CD61). Cette intégrine $\alpha_V\beta_3$ constitue une cible de choix pour l'imagerie en oncologie puisqu'elle est faiblement exprimée dans les tissus sains mais qu'elle est surexprimée lors de phénomènes d'angiogenèse, comme c'est le cas dans la plupart des cancers, par les néovaisseaux et en particulier par les cellules endothéliales.
On utilise avantageusement comme ligand de ciblage du ligand de ciblage amphiphile au moins un ligand de ciblage choisi parmi : les peptides (avantageusement de moins de 20 acides aminés, plus avantageusement de 5 à 10 acides aminés), les pseudopeptides, les peptidomimétiques, les acides aminés, les agents de ciblage d'intégrines (peptides et pseudopeptides, peptidomimétiques notamment), les glycoprotéines, les lectines, les dérivés ptéroïques ou aminoptéroïques, les dérivés de l'acide folique et antifolique, les anticorps ou fragments d'anticorps, les stéroïdes, les oligonucléotides, les séquences d'acide ribonucléique, les séquences d'acide désoxyribonucléique, les hormones, les protéines éventuellement recombinantes ou mutées, les mono- ou polysaccharides, les composés à squelette benzothiazole, benzofurane, styrylbenzoxazole/thiazole/imidazole/quinoline, styrylpiridine et composés dérivés, et leurs mélanges. Les peptides, les dérivés de l'acide folique et antifolique, les agents de ciblage d'intégrines (peptides et pseudopeptides, peptidomimétiques notamment), les agents de ciblage de récepteurs cellulaires ou d'enzymes (notamment de ciblage de kinases, notamment tyrosine kinase ; de métalloprotéases ; de caspases ...) sont particulièrement préférés.

On entend designer par "peptidomimétique", un composé qui n'est pas constitué d'un enchainement régulier d'acides aminés reliés entre eux par des liaisons peptidiques mais qui mime l'activité biologique d'un peptide.

On entend désigner par « pseudopeptide », n'importe quel analogue peptidique comportant au moins une modification de la liaison peptidique native -[CO-NH]-.

Par « dérivés ptéroïques ou aminoptéroïques », on entend des composés fonctionnalisés à partir de l'acide ptéroïque ou aminoptéroïque et ou modifiés par des groupements bioisostères comme décrit dans WO 2004/112839, WO 2007/042504, US 2005/0227985 et WO 2010/102238.

Par « dérivés de l'acide folique et antifolique », on entend des composés fonctionnalisés à partir de l'acide folique ou de l'acide antifolique et ou modifiés par des groupements bioisostères comme décrit dans WO 2004/112839, WO 2007/042504, US 2005/0227985 et WO 2010/102238. Ces dérivés incluent notamment l'acide folinique, l'acide ptéropolyglutamique et des ptéridines ligands du récepteur du folate comme les tetrahydropterines, les dihydrofolates, les tetrahydrofolates et leurs analogues deaza et dideaza. Ces analogues « deaza » et « dideaza » sont des analogues ayant un atome de carbone substitué par un ou deux atomes d'azote dans la structure de l'acide folique ou de l'acide antifolique. Par exemple, les analogues deaza incluent les analogues 1 -deaza, 3-deaza, 5-deaza, 8-deaza, and 10-deaza du folate. Les analogues dideaza incluent par exempleles analogues 1,5-dideaza, 5,10- dideaza, 8,10-dideaza, and 5,8-dideaza du folate. Comme autres dérivés de l'acide folique peuvent aussi être cités l'aminoptérine, l'amethoptérine (methotrexate), le N(10) -méthylfolate, le 2-deamino-hydroxyfolate, les analogues deaza comme le 1- deazaméthoptérine ou le 3-deazamethoptérine et l'acide 3',5'-dichloro-4-amino-4-deoxy-N(10)- methylptéroylglutamique (dichloromethotrexate).

Par « composés dérivés de styrylpiridine », on entend des composés obtenus à partir de la styrylpiridine et qui portent au moins une chaine fonctionnalisée sur l'un des deux noyaux aromatiques de celle-ci.

Selon des modes de réalisations avantageux, le ligand de ciblage (c'est-à-dire la partie permettant le ciblage) du ligand de ciblage amphiphile est choisi parmi la liste suivante (les documents et références entre parenthèses sont des exemples et non une liste limitative) :

1) Les ligands de ciblage ciblant des récepteurs VEGF et angiopoiétine (décrits dans WO 01/97850), les polymères tels que polyhystidine (US 6,372,194), les polypeptides ciblant la fibrine (WO 01/09188), les peptides de ciblage d'intégrines (WO 01/77145, WO 02/26776 pour alphaV-beta3, WO 02/081497, par exemple RGDWXE), les pseudopeptides et peptides de ciblage de métalloprotéases MMP (WO 03/062198, WO 01/60416), les peptides ciblant par exemple le récepteur KDR/Flk-I dont R-X-K-X-H et R-X-K-X-H, ou les récepteurs Tie-1 et 2 (WO 99/40947 par exemple), les glycosides de sialyl Lewis (WO 02/062810 et « Müller et al, Eur. J. Org. Chem, 2002, 3966-3973), les antioxydants tels que l'acide ascorbique (WO 02/40060), les ligands de ciblage de la tuftsine (par exemple US 6,524,554), de ciblage de récepteurs à protéine G GPCR en particulier la cholécystokinine (WO 02/094873), les associations entre antagoniste d'intégrine et mime de la guanidine (US 6,489,333), les quinolones ciblant alphav beta3 ou 5 (US 6,511,648), les benzodiazépines et analogues ciblant des intégrines (US 2002/0106325, WO 01/97861), les imidazoles et analogues (WO 01/98294), les peptides RGD (WO 01/10450), les anticorps ou fragments d'anticorps (FGF, TGFb, GV39, GV97, ELAM, VCAM, inductible par TNF ou IL (US 6,261,535), les molécule de ciblage modifiées par interaction avec la cible (US 5,707,605), les agents de ciblage de dépôts amyloïdes (WO 02/28441 par exemple), les peptides clivés cathepsines (WO 02/056670), les mitoxantrone ou quinone (US 6,410,695), les polypeptides ciblant des cellules épithéliales (US 6,391,280), les inhibiteurs de cystéines protéases (WO 99/54317), les ligands de ciblage décrits dans : US 6,491,893 (GCSF), US 2002/0128553, WO 02/054088, WO 02/32292, WO 02/38546, WO 03/006059, US 6,534,038, WO 01/77102, EP 1 121 377, Pharmacological Reviews (52, n°2, 179 ; facteurs de croissance PDGF, EGF, FGF...), Topics in Current Chemistry (222, W.Krause, Springer), Bioorganic & Medicinal Chemistry (11, 2003, 1319-1341 ; dérivés tétrahydrobenzazépinones ciblant alphaV-beta3).

2) Les inhibiteurs d'angiogenèse, notamment ceux testés en essais cliniques ou déjà commercialisés, notamment :

- les inhibiteurs d'angiogenèse impliquant des récepteurs FGFR ou VEGFR tels que SU101, SU5416, SU6668, ZD4190, PTK787, ZK225846, des composés azacycles (WO 02/44156, WO 02/059110) ;
- les inhibiteurs d'angiogenèse impliquant des MMP tels que le BB25-16 (marimastat), le AG3340 (prinomastat), le solimastat, le BAY12-9566, le BMS275291, le metastat, le neovastat ;
- les inhibiteurs d'angiogenèse impliquant des intégrines tels que le SM256, le SG545, des molécules d'adhésion bloquant le EC-ECM (tels que le EMD 121-974, ou la vitaxine) ;
- des médicaments à mécanisme d'action anti-angiogénique plus indirect tels que le carboxiamidotriazole, le TNP470, la squalamine, le ZD0101 ;
- les inhibiteurs décrits dans le document WO 99/40947, les anticorps monoclonaux très sélectifs pour la liaison au récepteur KDR, les analogues de la somatostatine (WO 94/00489), les peptides de liaison à la sélectine (WO 94/05269), des facteurs de croissance (VEGF, EGF, PDGF, TNF, MCSF, interleukines); des ligands de ciblage de ciblage de VEGF décrits dans Nuclear Medicine Communications, 1999, 20 ;

- les peptides inhibiteurs du document WO 02/066512.

3) Des ligands de ciblage capables de cibler des récepteurs : CD36, EPAS-1, ARNT, NHE3, Tie-1, 1/KDR, Flt-1, Tek, neuropiline-1, endogline, pléientropine, endosialine, Axl., alPi, a2ssl, a4P1, a5pl, eph B4 (éphrine), récepteur laminine A, récepteur neutrophiline 65, récepteur leptine OB-RP, récepteur chimiokine CXCR-4 (et autres récepteurs cités dans le document WO99/40947), LHRH, bombésine/GRP, récepteurs gastrine, VIP, CCK.
4) Des ligands de ciblage de type inhibiteurs de tyrosine kinase.
5) Les inhibiteurs du récepteur GPIIb/IIIa connus choisis parmi : (1) le fragment fab d'un anticorps monoclonal du récepteur GPIIb/IIIa, Abciximab, (2) les petites molécules peptidiques et peptidomimétiques injectées en intraveineuse telles que l'eptifibatide et le tirofiban.
6) Des peptides antagonistes de récepteurs au fibrinogène (EP 0 425 212), des peptides ligands de récepteurs IIb/IIIa, des ligands du fibrinogène, des ligands de la thrombine, des peptides capables de cibler la plaque d'athérome, les plaquettes, la fibrine, des peptides à base d'hirudine, des dérivés à base de guanine ciblant le récepteur IIb/IIIa.
7) D'autres ligands de ciblage ou fragments biologiquement actifs de ligands de ciblage connus de l'homme du métier comme médicaments, à action anti-thrombotique, anti agrégation plaquettaire, antiathérosclérotique, antiresténotique, anticoagulante.
8) D'autres ligands de ciblage ou fragments biologiquement actifs de ligands de ciblage ciblant alpha V beta3, décrits en association avec des DOTA dans le brevet US 6 537 520, choisis parmi les suivants : mitomycine, tretinoine, ribomustine, gemcitabine, vincristine, etoposide, cladribine, mitobronitol, methotrexate, doxorubicine, carboquone, pentostatine, nitracrine, zinostatine, cetrorelix, letrozole, raltitrexed, daunorubicine, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoine, streptozocine, nimustine, vindesine, flutamide, drogenil, butocin, carmofur, razoxane, sizofilan, carboplatine, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, leutinizing hormone releasing factor.
9) certains ligands de ciblage ciblant des types particuliers de cancers, par exemple des peptides ciblant le récepteur ST associé au cancer colorectal, ou le récepteur tachykinine.
10) des ligands de ciblage utilisant des composés de type phosphines.
11) des ligands de ciblage de ciblage de P-sélectine, E-sélectine ; par exemple, le peptide de 8 acides aminés décrit par Morikawa et al, 1996, 951, ainsi que différents sucres.
12) l'annexine V ou des ligands de ciblage ciblant les processus apoptotiques.
13) tout peptide obtenu par des technologies de ciblage telles que le phage display, modifié éventuellement par des acides aminés non naturels (http//chemlibrary.bri.nrc.ca), par exemple des peptides issus de banques phage display : RGD, NGR, CRRETAWAC, KGD, RGD-4C, XXXY*XXX, RPLPP, APPLPPR.
14) d'autres ligands de ciblage peptidiques connus de ciblage de plaques d'athérome, cités notamment dans le document WO 2003/014145 et notamment VCAM
15) des vitamines.
16) des ligands de récepteurs hormonaux dont les hormones et les stéroïdes.
17) des ligands de ciblage ciblant des récepteurs opioïdes.
18) des ligands de ciblage ciblant des récepteurs TKI.
19) des antagonistes LB4 et VnR.
20) des composés nitriimidazoles et benzylguanidines.
21) des ligands de ciblage rappelés dans Topics in Current Chemistry, vol.222, 260-274, Fundamentals of Receptor-based Diagnostic Metallopharmaceuticals, notamment :

- des ligands de ciblage de ciblage de récepteurs peptidiques surexprimés dans les tumeurs (récepteurs LHRH, bombésine/GRP, récepteurs VIP, récepteurs CCK, récepteurs tachykinine par exemple), notamment les analogues de somatostatine ou de bombésine, des peptides dérivés octréotide éventuellement glycosylés, les peptides VIP, les alpha-MSH, les peptides CCK-B
- des peptides choisis parmi : des peptides cycliques RGD, fibrine-chaîne alpha, CSVTCR, tuftsine, fMLF, YIGSR (récepteur : laminine).

22) des oligosaccharides, des polysaccharides et des dérivés d'oses, des dérivés ciblant les récepteurs Glut (récepteurs d'oses).
23) des ligands de ciblage utilisés pour des produits de type smart.
24) des marqueurs de la viabilité myocardique (tétrofosmine et hexakis2méthoxy-2méthylpropylisonitrile).

25) des traceurs du métabolisme des sucres et des graisses.

26) des ligands de récepteurs de neurotransmetteurs (récepteurs D, 5HT, Ach, GABA, NA).

27) des oligonucléotides.

28) le facteur tissulaire

29) des ligands de ciblage décrits dans WO 03/20701, en particulier le PK11195 ligand du récepteur périphérique aux benzodiazépines.

30) des peptides liant la fibrine, notamment les séquences peptidiques décrites dans WO 03/11115.

31) des inhibiteurs d'agrégation de plaques amyloïdes (décrits par exemple dans WO 02/085903).

32) des pharmacophores composés de ciblage de la maladie d'Alzheimer, en particulier les composés comprenant les squelettes de type benzothiazole, benzofuranes, styrylbenzoxazoles/thiazoles/imidazoles/quinoline, styrylpiridines.

33) des pharmacophores composés de ciblage obtenus à partir de squelettes chimiques à activité pharmacologique décrits dans US2007098631, notamment les formules des pages 4 à 10 et pages 13 -14, notamment les composés du tableau page 4 dans la colonne intitulée « scaffolds and derivatives » : biphenyl; arylpiperidine; arylpiperazine; 1,4 dihydropyridine dihydropyrimidone; 1,4 benzodiazepine-2-one; 1,5 benzodiazepine-2-one; 1,4-benzodiazepine-2,5 diones; pyrrolo2,1-c 1,4 benzodiazepines 5,11 diones; 1,4 benzothiazepine-5-ones; 5,11-dihydro-benzo pyrido 3,2b 1,4 diazepine-6-ones benzopyrane; chromone; benzopyranone; coumarine, pyranocoumarine; benzopiperazinones; quinazolinone; quinazolindione; quinoxalinone; imidazoquinoxaline; indole; benzimidazole, benzofurane, benzothiophene.

34) des ligands de ciblage d'intégrines, notamment des composés non peptidiques mimétiques de peptides RGD, et en particulier des composés tetrahydro naphtyridine décrits par exemple dans : J Med. Chem., 2003, 46, 4790-4798, Bioorg. Med. Chem. Letters, 2004, 14, 4515-4518, Bioorg. Med. Chem. Letters, 2005, 15, 1647-1650.

35) des ligands de ciblage de MUC5AC, notamment des fragments d'anticorps, des peptides et des composés non peptidiques mimétiques de peptides.

36) des ligands d'une cible associée à l'angiogenèse, notamment d'une cible choisie parmi 4N1K, AGF ("angiopoietin-related growth factor" en anglais), Angiogénine (ANG), Angiopoïetine-1, Angiopoïetine -2, Angiostatine, ARP4 ("angiopoietin-related protein 4" en anglais), bFGF ("basic fibroblast growth factor" en anglais), CD31 (PECAM-1), CD34, CD97, CD146 (MUC18), CECs ("circulating endothelial cells" en anglais), CEPs ("circulating endothelial precursor cells" en anglais), Collagénase-1 (C1), COX-2 (Cyclooxygénase-2), E7820, EG-1 ("endothelial-derived gene-1" en anglais), Extra-Domaine B (ED-B) de la Fibronectine, Endogline (CD105), ESAF ("Endothelial cell stimulating angiogenesis factor" en anglais), Facteur VIII (facteur anti-hémophilique A), Flt-1 ("Fms-like tyrosine kinase 1" en anglais), Intégrine $\alpha1\alpha2$, Intégrine $\alpha2\beta1$, Intégrine $\alpha$ $3\beta1$, Intégrine $\alpha5\beta1$, Intégrine $\alpha v\beta3$, Intégrine $\alpha6\beta4$, Intégrine $\alpha9\beta1$, Intégrine-$\beta1$, KDR, N-Cadhérine, Nestine, NG2 protéoglycane, PSMA ("prostate-specific membrane antigen" en anglais), PV-1 ("Plasmalemmal vesicle associated protein-1" en anglais), S100A13, Syndécane-1, T-Cadhérine, TEM-5 ("Tumor endothelial marker 5" en anglais), TEM-8 ("Tumour endothelial marker-8" en anglais), Thrombospondine-1 (TSP1), Thrombospondine-2 (TSP2), Thy-1, Tie-1, Tie-2, Tn-C (Ténascine-C), TP (Thymidine phosphorylase), VCAM-1 ("vascular cell adhésion molecule-1" en anglais), VE-cadhérine, VEGF, VWF ("von Willebrand Factor" en anglais) et les inhibiteurs d'angiogenèse rappelés au paragraphe 2) ci-dessus.

En particulier, pour ces composés tetrahydro naphtyridine, tout composé de naphtyridine connu de l'art antérieur peut être utilisé (notamment ceux de WO 2009/114776), l'utilisation des composés naphtyridine comme ligand de ciblage pour imagerie médicale étant décrite dans WO 2007/042506 page 13 lignes 30-34.

Le ligand de ciblage du ligand de ciblage amphiphile préférentiel selon l'invention est choisi parmi les peptides et/ou les peptidomimétiques et/ou les ligands de ciblage des intégrines (par exemple de l'intégrine $\alpha V\beta3$). De façon avantageuse, le ligand de ciblage du ligand de ciblage amphiphile est un peptidomimétique. De façon très avantageuse, le ligand de ciblage du ligand de ciblage amphiphile est un composé peptidomimétique de ciblage d'intégrines et en particulier, un composé peptidomimétique de ciblage d'intégrines choisi parmi les composés comprenant un groupe naphtyridine. De façon encore plus avantageuse, le ligand de ciblage amphiphile est un composé peptidomimétique de ciblage d'intégrines de formule :

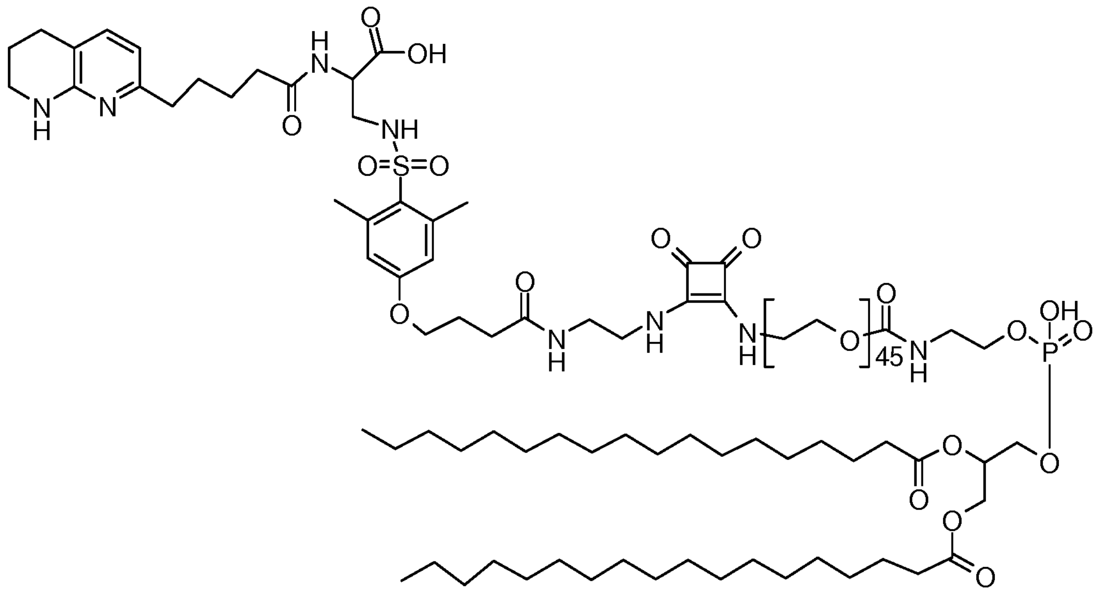

ou un de ses sels, par exemple sous forme de phosphate d'ammonium.

Ce peptidomimétique du RGD a comme propriété de cibler l'intégrine $\alpha V\beta 3$.

Les ligands de ciblage du ligand de ciblage amphiphile pour la reconnaissance de la cible en milieu biologique se situent essentiellement du côté de la surface externe des nanogouttelettes, le groupe lipophile du ligand de ciblage amphiphile s'insérant dans la couche de tensioactif.

Le ligand de ciblage amphiphile s'écrit avantageusement sous la forme : Bio - L - Lipo dans laquelle :

- Bio est le ligand de ciblage (notamment un des ligands de ciblage mentionné ci-dessus) localisé à la surface externe des nanogouttelettes,
- Lipo est un groupe lipophile s'insérant au sein de la couche de tensioactif,
- L est un groupe de liaison reliant Bio et Lipo, avantageusement choisi parmi :

    ▪ une simple liaison, squarate, C1-6 alkylène, PEG, par exemple $CH_2$-$(CH_2$-O-$CH_2)_k$-$CH_2$ avec k=1 à 10, $(CH_2)_3$-NH, NH-$(CH_2)_2$-NH, NH-$(CH_2)_3$-NH, , $(CH_2)_n$, $(CH_2)_n$-CO-, -$(CH_2)_n$NH-CO- avec n = 2 à 10 , $(CH_2CH_2O)_q(CH_2)_r$-CO- , $(CH_2CH_2O)_q(CH_2)_r$-NH-CO-avec q = 1-10 et r=2-10, $(CH_2)_n$-CONH-, $(CH_2)_n$-CONH-PEG, $(CH_2)_n$-NH-, -OOC-$CH_2$-O-$(CH_2)_2$-O-$(CH_2)_2$-O-$CH_2$-COO- ; -OOC-$(CH_2)_2$-$CO_2$-$(CH_2)_2$-OCO-$(CH_2)_2$-COO- ; -OOC-CH(OH)-CH(OH)-COO-; -OOC-$(CH_2)_n$-COO-; -NH-$(CH_2)_n$-NH-, avec n=0-20; NH-$(CH_2)_n$-$CO_2$; NH-$CH_2$-$(CH_2$-O-$CH_2)_n$-$CO_2$ avec n=1 à 10,

    ▪ P1-I-P2, identiques ou différents, P1 et P2 étant indépendamment choisis parmi O, S, NH, une liaison simple, $CO_2$, NHCO, CONH, NHCONH, NHCSNH, $SO_2$NH-, $NHSO_2$-, squarate

  avec I = alkylène, alkoxyalkylène, polyalkoxyalkylène (notamment PEG), alkylène interrompu par un ou plusieurs squarates, par un ou plusieurs aryles, avantageusement phényles, ou par un ou plusieurs groupes choisis parmi -NH-, -O-, -CO-, -NH(CO)-, - (CO)NH-, -O(CO)-, et -(OC)O-, alkenylène ou alkynylène, lesdits alkenylène ou alkynylène étant éventuellement interrompus par un ou plusieurs groupes choisis parmi -NH-, -O-, -CO-, -NH(CO)-, -(CO)NH-, -O(CO)-, ou -(OC)O-.

Les liaisons covalentes L ou I entre Bio et Lipo sont avantageusement de type -CONH-, - COO-, -NHCO-, -OCO-, -NH-CS-NH-, -C-S-, -N-NH-CO-, -CO- NH-N-, -$CH_2$-NH-, -N-$CH_2$-, -N-CS-N-, -CO-$CH_2$-S-, -N-CO-$CH_2$-S-, -N-CO-$CH_2$-$CH_2$-S-, -CH=NH-NH-, -NH-NH=CH-, - CH=N-O-, -O-N=CH- ou répondant aux formules suivantes :

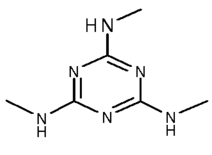 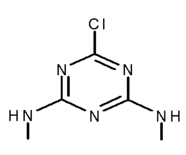 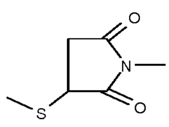 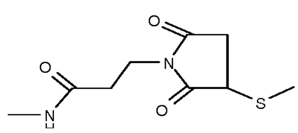

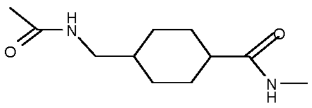
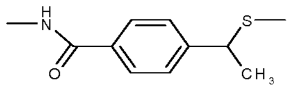
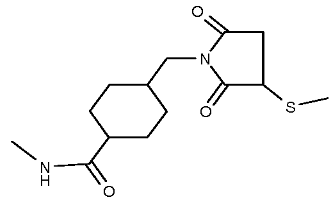

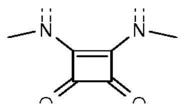

Lorsque les groupes divalents mentionnés ci-dessus comme groupes L ou I possibles ne sont pas symétriques, il est sous-entendu que le groupe Bio- ou Bio-P1- peut être greffé à droite ou à gauche dudit groupement covalent. Par exemple, lorsqu'on écrit L représente un groupe -COO-, le ligand de ciblage peut être Bio-COO-Lipo ou Lipo-COO-Bio. On présente quelques exemples de ligands de ciblage amphiphiles (ci-après : peptides, dérivés de l'acide folique, dérivés naphtyridine), rendus amphiphiles pour l'ancrage à la surface externe de la nanogouttelette.

La demande présente des exemples illustratifs de leur synthèse.

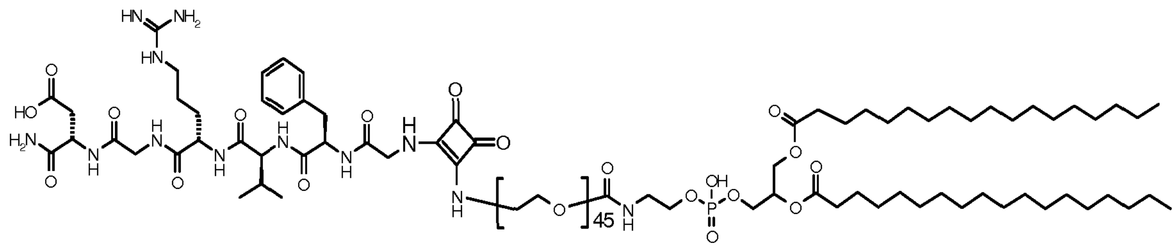

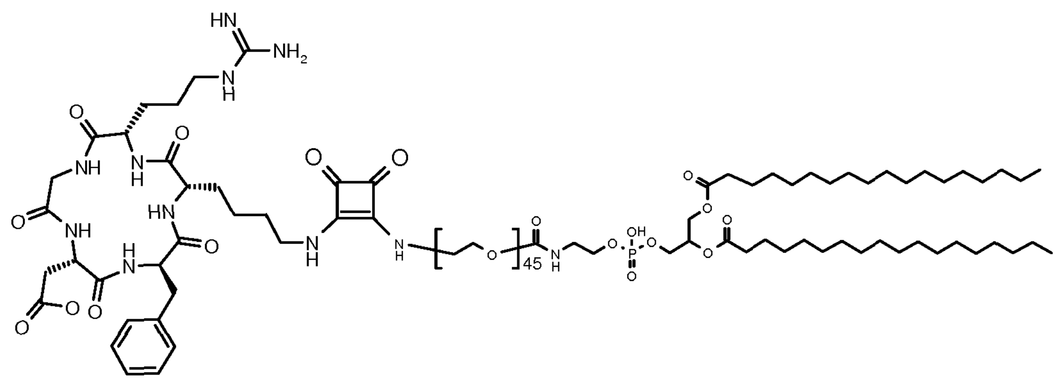

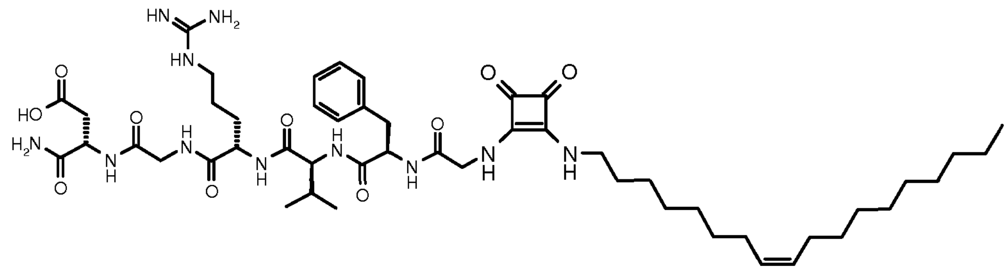

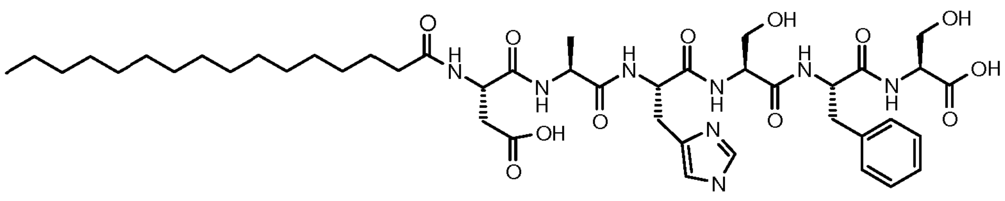

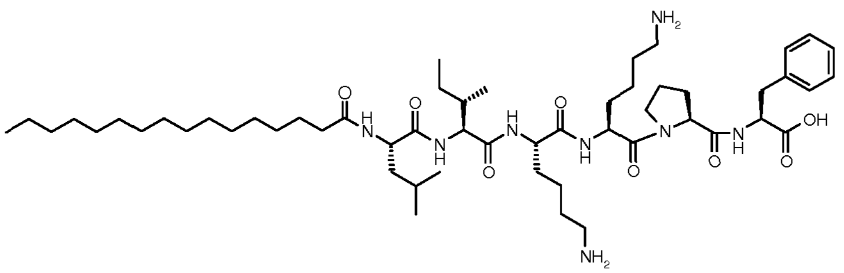

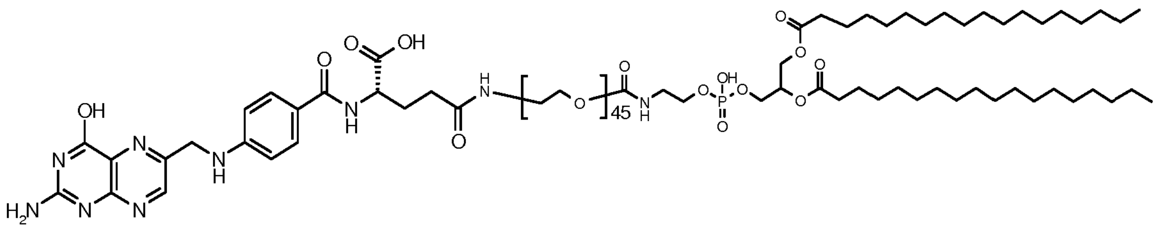

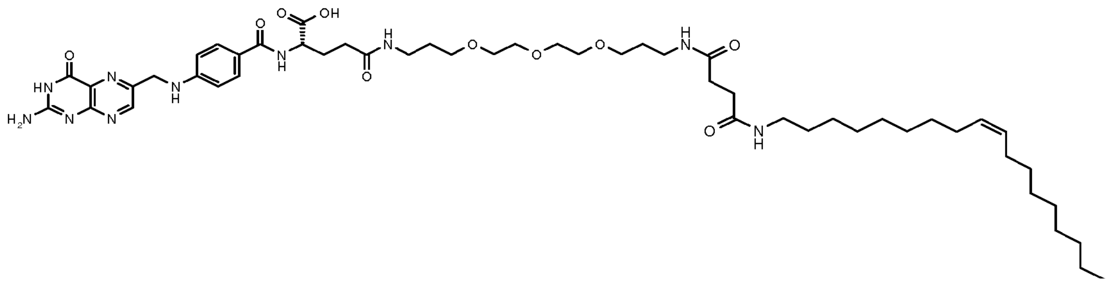

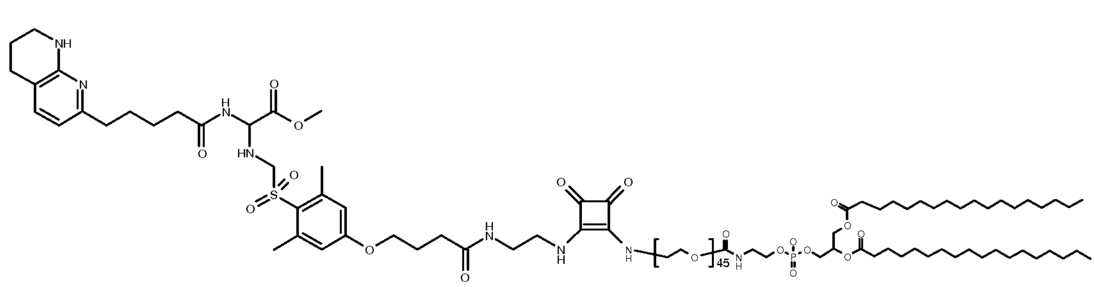

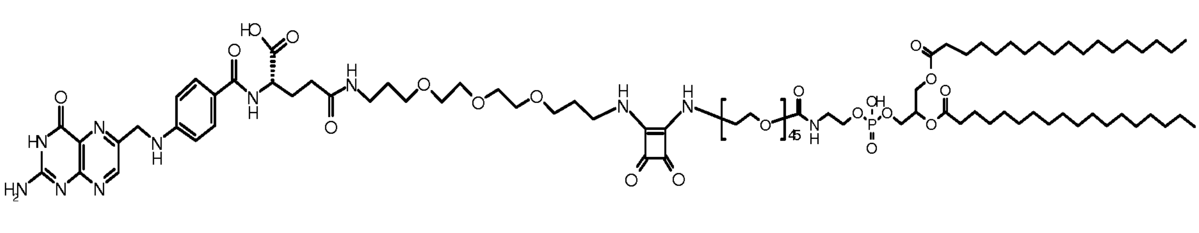

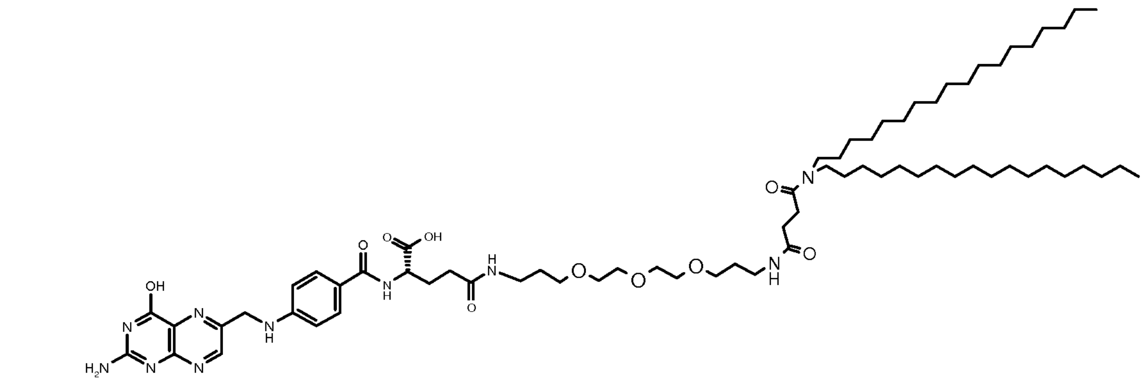

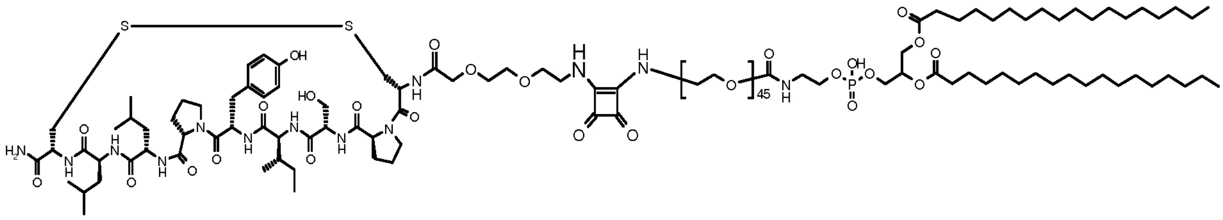

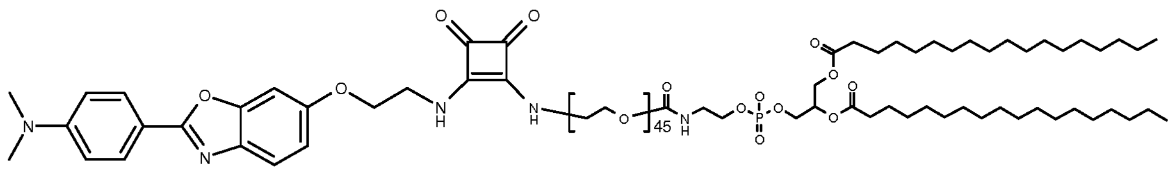

Comme expliqué dans la demande, les compositions sont essentiellement utilisées pour l'imagerie diagnostique. On peut toutefois préparer des nanoémulsions comprenant en outre des ligands comprenant un agent thérapeutique. Les nanogouttelettes comprendront alors d'une part un ligand de ciblage amphiphile pour atteindre la cible biologique (la zone pathologique), et d'autre part un ligand comprenant un agent thérapeutique pour le traitement thérapeutique. L'invention concerne ainsi aussi les compositions décrites précédemment, comprenant un agent thérapeutique, pour leur utilisation pour le traitement de maladies, notamment cancéreuses, neurodégénératives, vasculaires.

Selon des modes de réalisation, le mélange de tensioactifs comprend en outre au moins un agent de furtivité amphiphile, avantageusement un dérivé PEG, un dérivé de ganglioside (résidus osidiques estérifiés typiquement par l'acide sialique ou le NAC), un polysaccharide (notamment le dextran ou l'un de ses dérivés connus). Ces agents de furtivité sont intégrés sans altérer l'affinité de la nanogouttelette pour la cible biologique.

**Procédé de préparation d'une nanoémulsion selon l'invention**

Selon un autre aspect, l'invention concerne un procédé de préparation d'une composition telle que définie ci-dessus, comprenant les étapes de:

a) Solubilisation des particules magnétiques (p) à base d'un composé de fer, recouvertes par un ou plusieurs acides gras en C8-C22, dans une huile comprenant au moins 70%, de préférence au moins 80, 90, 95, 97 % en poids de glycérides d'acides gras saturés en C6-C18, avantageusement en C6-C14, et très avantageusement en C6-C10 pour former une phase lipidique,
b) Mélange de la phase lipidique obtenue à l'étape a) et d'une phase aqueuse comprenant le mélange des tensioactifs, de manière à former des nanogouttelettes,
c) récupération de la nanoémulsion obtenue.

De préférence, la solubilisation de l'étape a) est totale. Par « solubilisation totale », on entend qu'après mélange de l'huile et des particules magnétiques (p) à base d'un composé de fer, préalablement recouvertes par un ou plusieurs acides gras en C8-C22 il y ait une absence d'agrégat visible à l'oeil nu dans la phase lipidique formée.

Les particules magnétiques (p) à base de composé de fer sont recouvertes par un ou plusieurs acides gras

en C8-C22, par exemple, par dilution des particules magnétiques dans une solution alcaline (solution de soude (NaOH) à $5.10^{-3}$ M par exemple) et ajout d'un large excès (50 à 300, préférentiellement 50 à 150 équivalents) d'un ou plusieurs acides gras en C8-C22, tels que décrits ci-dessus, conduisant à une précipitation des particules magnétiques.

L'étape a) de solubilisation, dans l'huile, des particules magnétiques (p) est généralement réalisée par agitation à une température supérieure à 60°C, préférentiellement supérieure à 80°C, encore plus préférentiellement supérieure à 90°C, voir supérieure à 100°C.

L'agitation durant l'étape de solubilisation a) peut être réalisée pendant 10 à 40 heures, préférentiellement 15 à 30 heures, encore plus préférentiellement 24 heures.

Le mélange de tensioactifs (lipide amphiphile, ligand de ciblage amphiphile et lipide pégylé le cas échéant) est généralement ajouté dans la phase aqueuse (définie ci-dessus) par dispersion, par exemple à l'aide d'ultrasons, autrement dit par sonication.

Lors de l'étape b), la nanoémulsion est avantageusement obtenue par émulsification dans un microfluidiseur. La nanoémulsion ainsi obtenue est alors utilisée pour l'administration au patient, le cas échéant après incorporation d'additifs pharmaceutiques variés. Elle peut également être filtrée. Les nanoémulsions obtenues peuvent être lyophilisées avec le cas échéant utilisation d'agents anti agglutination.

On obtient typiquement les caractéristiques suivantes (obtenues grâce aux méthodes analytiques précisées au début de la partie Exemple ci-après), pouvant varier selon les compositions précises des émulsions et leur procédé de préparation :

- diamètre hydrodynamique (Z ave): 150 à 220 nm, préférentiellement 185 à 200 nm, encore plus préférentiellement 190 à 200 nm.
- index de polydispersité (Poly $\sigma$): 0,01 à 0,1, préférentiellement 0,03 à 0,08, encore plus préférentiellement 0,04 à 0,06.
- concentration en fer (mmoles/litre de composition) : 100 à 300 préférentiellement 120 à 200, encore plus préférentiellement 140 à 160.
- concentration en gouttelettes dans la nanoémulsion: 80 à 200 nM, préférentiellement 90 à 120 nM.
- relaxivité 60 MHz (milieu eau / 37°C)
    - r1 ($mM^{-1}$ (Fe) $s^{-1}$) : 0,5 à 4, de préférence 0,8 à 2, encore plus préférentiellement 1.
    - r2 ($mM^{-1}$ (Fe) $s^{-1}$) : 180 à 250, préférentiellement 190 à 220, encore plus préférentiellement 210.
- Osmolalité : 280 - 320 mOsm/kg.
- stabilité en solution pharmaceutique : supérieure à 6 mois.
- stabilité dans le Seronorm™ (stabilité biologique): supérieure à 24h.
- nombre de particules magnétiques par gouttelette de nanoémulsion : plus de 90, préférentiellement plus de 100, encore plus préférentiellement plus de 140.
- nombre de ligands de ciblage amphiphile par gouttelette de nanoémulsion : 100 à 5000, notamment 500 à 4000, avantageusement 1800 à 3500, encore plus avantageusement 2500 à 3500.

Au global, les nanoémulsions pour l'IRM obtenues :

- ont une efficacité supérieure à celles des émulsions actuellement connues en terme de signal pour l'imagerie clinique (IRM en particulier) chez le patient,
- ont une affinité vis-à-vis des récepteurs qu'elles ciblent, supérieure à celle d'autres émulsions non conformes à la présente invention,
- sont capables d'incorporer des ligands de ciblage de zones pathologiques à la surface des nanogouttelettes, en quantité appropriée et sans perte d'affinité gênante avec leur cible biologique,
- sont suffisamment stables chimiquement pour être produites et conservées une longue durée (plusieurs mois à plusieurs années), en particulier sans problème de coalescence entre-elles des gouttelettes,
- sont suffisamment stables *in vivo* pour ne pas être dégradées,
- sont adaptées sur le plan de la pharmacocinétique,
- sont réalisées avec des huiles répertoriées dans la pharmacopée.

**Produit de contraste comprenant la composition de nanoémulsion selon l'invention**

Selon un autre aspect, l'invention concerne un produit de contraste, de préférence pour IRM, comprenant la composition de nanoémulsion telle que décrite ci-dessus.

Le produit de contraste est administré de préférence en intravasculaire, selon le patient examiné, par exemple, pour une composition présentant une concentration en Fer de 145 mM, à raison de 50 à 180, préférentiellement 80 à

120, très préférentiellement 90 à 110, encore plus préférentiellement 100 μmoles de fer par kg de patient, ce qui correspond à des volumes de 0,34 à 1,24, préférentiellement 0,55 à 0,83, encore plus préférentiellement 0,7 ml de produit de contraste par kg de patient.

Le produit de contraste peut être formulé à l'aide d'additifs connus rappelés, par exemple, dans US 6,010,682, notamment pour une administration par injection intraveineuse. Il peut notamment comprendre des agents épaississants, des saccharides ou polysaccharides, le glycérol, le dextrose, le chlorure de sodium et/ou des antimicrobiens.

**Utilisation des compositions de nanoémulsions et produits de contraste selon l'invention**

L'invention concerne aussi les compositions décrites précédemment pour leur utilisation pour le diagnostic de maladies, notamment cancéreuses, inflammatoires, neurodégénératives ou vasculaires, notamment cardiovasculaires.

L'invention concerne une méthode d'imagerie du corps entier ou d'une partie du corps d'un individu comprenant comprenant une étape d'obtention d'une ou plusieurs images du corps entier ou d'une partie du corps d'un individu par une technique d'imagerie médicale, dans laquelle ledit corps entier ou ladite partie du corps de l'individu comprend la composition définie ci-dessus ou le produit de contraste défini ci-dessus (de préférence dans une quantité efficace) et dans laquelle la ou les image(s) sont associée(s) aux particules magnétiques à base d'un composé du fer contenues dans la composition définie ci-dessus ou dans le produit de contraste défini ci-dessus.

Selon un mode de réalisation, la méthode d'imagerie selon l'invention n'inclut pas d'étape d'injection ou d'administration invasive de la composition ou du produit de contraste à l'individu.

Selon un autre mode de réalisation, la méthode d'imagerie selon l'invention comprend une étape préalable d'injection ou d'administration de la composition ou du produit de contraste à l'individu, de préférence une injection par voie intravasculaire.

Cette méthode permet une acquisition dynamique en visualisant les phases de rehaussement au cours du temps de la zone d'intérêt et de wash out.

Les données cliniques obtenues, pourront permettre d'aider un médecin à déterminer si le patient doit ou non recevoir un traitement thérapeutique donné, par exemple un traitement anti-angiogénique.

Ainsi, l'invention concerne également :

- la composition dont le ligand de ciblage du ligand de ciblage amphiphile est un ligand d'une cible associée à l'angiogenèse telle que définie ci-dessus, ou
- le produit de contraste comprenant une composition dont le ligand de ciblage du ligand de ciblage amphiphile est un ligand d'une cible associée à l'angiogenèse tel que défini ci-dessus,

destiné(e) à être utilisée dans une méthode d'évaluation « in vivo » de l'angiogenèse chez un individu comprenant les étapes consistant à :

a) obtenir une ou plusieurs images du corps entier ou d'une partie du corps d'un individu par une technique d'imagerie médicale, dans laquelle ledit corps entier ou ladite partie du corps de l'individu comprend :

- la composition dont le ligand de ciblage du ligand de ciblage amphiphile est un ligand d'une cible associée à l'angiogenèse telle que définie ci-dessus, ou
- le produit de contraste comprenant une composition dont le ligand de ciblage du ligand de ciblage amphiphile est un ligand d'une cible associée à l'angiogenèse tel que défini ci-dessus

et dans laquelle la ou les image(s) est(sont) associée(s) aux particules magnétiques à base d'un composé du fer contenues dans ladite composition ou dans ledit produit de contraste;

b) évaluer l'angiogenèse dans le corps entier ou la partie du corps de l'individu à partir de la ou des images obtenues à l'étape a).

La méthode d'imagerie définie ci-dessus peut aussi permettre l'évaluation de l'efficacité d'un traitement, notamment d'un traitement anti-angiogénique, préférentiellement d'un traitement anti-cancéreux et de manière plus préférentielle d'un traitement anti-cancéreux anti-angiogénique (du type Avastin® par exemple).

Ainsi, l'invention concerne également :

- la composition dont le ligand de ciblage du ligand de ciblage amphiphile est un ligand d'une cible associée à l'angiogenèse telle que définie ci-dessus, ou
- le produit de contraste comprenant une composition dont le ligand de ciblage du ligand de ciblage amphiphile est un ligand d'une cible associée à l'angiogenèse tel que défini ci-dessus,

pour son utilisation dans une méthode d'évaluation de l'efficacité d'un traitement anti-angiogénique comprenant les étapes consistant à :

a) évaluer l'angiogénèse chez un individu ayant reçu un traitement anti-angiogénique, par la méthode définie ci-dessus ;
b) comparer l'angiogenèse évaluée selon l'étape a) avec une référence de l'angiogénèse chez l'individu avant le traitement anti-angiogénique.

La référence peut être le niveau préalablement à tout traitement anti-angiogénique chez l'individu (niveau de base) ou à une étape antérieure du traitement thérapeutique.
Le traitement anti-angiogénique est considéré comme efficace si une diminution ou un maintien de l'angiogenèse est observé par rapport à la référence, inefficace si une augmentation de angiogenèse est observée par rapport à la référence.
La méthode, selon un mode de réalisation, n'inclut pas d'étape d'administration du traitement anti-angiogénique.
Dans un mode de réalisation, la méthode d'évaluation de l'efficacité d'un traitement anti-angiogénique définie ci-dessus comprend :

a) l'évaluation de l'angiogenèse chez un individu pour déterminer niveau de base par la méthode définie ci-dessus,
b) l'administration d'un traitement anti-angiogénique audit individu, de préférence par injection par voie intravasculaire,
c) l'évaluation de l'angiogenèse après ledit traitement chez l'individu pour déterminer niveau après traitement par la méthode définie ci-dessus,
d) la comparaison du niveau après traitement et du niveau de base.

Dans les méthodes définies ci-dessus, les images sont de préférence obtenues par Imagerie par Résonance Magnétique (ou IRM) ou imagerie MPI (Magnetic Particle Imaging).
Par « quantité efficace », on entend une quantité de composition de nanoémulsions ou de produit de contraste comprenant cette composition de nanoémulsion, qui permet d'obtenir les images par la technique d'imagerie médicale utilisée.
Les exemples figurant ci-après sont présentés à titre illustratif de l'invention.

**Description des figures:**

**Figure 1 :** schéma de la structure d'un exemple de nanoémulsion selon l'invention.

**1** désigne une particule magnétique (p) à base d'un composé du fer et recouverte par un ou plusieurs acides gras en C8-C22.
**2** désigne la phase lipidique comprenant l'huile selon l'invention.
**3** désigne un lipide amphiphile.
**4** désigne un lipide pégylé.
**5** désigne un ligand de ciblage amphiphile (ou « biovecteur » amphiphile). **3, 4** et **5** désignent les composés utilisés en tant que tensioactif dans la nanoémulsion.

**Figure 2 :** Valeur du R2* moyen de la tumeur extrait de la carte R2*, une heure post-injection.

En grisé hachuré, apparaissent les valeurs moyennes du R2* obtenues et leurs écart-types pour les tumeurs une heure après injection, soit de l'émulsion E1, soit de son contrôle E1T.
En blanc, apparaissent les valeurs moyennes du R2* obtenues et leurs écart-types dans la zone contro latérale à la tumeur.
Les triangles noirs correspondent aux points R2* des tumeurs.

**EXEMPLES :**

**Généralités**

Dans ce qui suit, les abréviations M, M théorique, N et M/z, $ES^+$, $ES^-$, kD et CCM, Z ave, XRD, Poly $\sigma$, TA,

$m_{obt}$ et Rdt ont les significations suivantes :

M : concentration molaire (mole/l).
M théorique : masse moléculaire théorique.
N : normalité.
M/z : masse sur charge déterminé par spectrométrie de masse.
$ES^+$ : électrospray mode positif.
$ES^-$ : électrospray mode négatif.
kD : unité de masse moléculaire (kiloDalton)
CCM : Chromatographie sur Couche Mince
Z ave : diamètre hydrodynamique mesuré par PCS en mode unimodal
XRD = diamètre de la particule (p) estimé par diffractométrie de rayons X
Poly $\sigma$ : polydispersité mesurée par PCS.
TA : température ambiante
$m_{obt}$ : masse de produit obtenu
Rdt : rendement

Dosage du Fer total:

La concentration en fer de la composition de nanoémulsion est mesurée par spectrométrie d'émission atomique à plasma à couplage inductif (ICP-AES, *Inductively Coupled Plasma Atomic Emission Spectroscopy*), après minéralisation dans $HNO_3$ 65% (équipement Optima™ 8300DV de Perkin Elmer ou équivalent).

Taille des particules/gouttelettes :

Diamètre hydrodynamique des gouttelettes de nanoémulsion (Z ave) :

Déterminé par PCS (appareil Malvern Nano ZS, laser 633 nm à 173°) sur un échantillon dilué à ~ 1 millimolaire avec de l'eau PPI filtrée sur 0.22 μm.
PCS = Photon Corrélation Spectroscopy = Technique par Diffusion de Lumière Dynamique - Référence : R. Pecora dans J. of Nano. Res. (2000), 2, p 123-131.

Diamètre de la particule magnétique (p)

Déterminé par diffractométrie de rayons X (ou XRD pour « X Ray diffraction ») à l'aide d'un détecteur courbe INEL CPS-120. Le logiciel de traitement et mesure est Fityk®, les pics sont modélisés selon des "split Pearson VII".

Analyses structurales :

Pour l'analyse qualitative, par spectrométrie de masse MALDI-TOF : spectromètre de masse couplant une source d'ionisation laser assistée par une matrice (MALDI, Matrix-Assisted Laser Desorption/Ionisation) et un analyseur à temps de vol (TOF, Time-of-Flight Mass Spectrometry). L'échantillon est d'abord dilué au 1/50ème dans du tétrahydrofurane (THF), puis le mélange est dilué au 1/10ème dans la matrice (DHB=acide 2,5-dihydroxybenzoïque à 20 mg/mL dans THF) avant analyse par le Voyager DE STR de PerSeptive Biosystems ou équivalent.
Pour l'analyse quantitative, par LC/UV/Corona (chromatographie liquide sur instrument UltiMate3000 (Dionex) couplé au détecteur Corona (Dionex)).

Mesures de relaxivité :

Les temps de relaxation T1 et T2 ont été déterminés par des procédures standards sur un appareil Minispec 120 (Bruker) à 20 Mhz (0,47T) et 37°C. Le temps de relaxation longitudinal T1 a été mesuré en utilisant une séquence d'Inversion Récupération et le temps de relaxation transverse T2 a été mesuré par une technique CPMG.
Les vitesses de relaxation R1 (=1/T1) et R2 (=1/T2) ont été calculées pour différentes concentrations en métal total (variant de $0,1.10^{-3}$ à $1.10^{-3}$ mole/L) en solution aqueuse à 37°C. La corrélation entre R1 ou R2 en fonction de la concentration est linéaire, et la pente représente la relaxivité r1 (R1/C) ou r2 (R2/C) exprimées en (1 / seconde) x (1/mMole/L) soit ($mM^{-1}.s^{-1}$).

Dosage du ligand de ciblage amphiphile RGD :

Le dosage du ligand de ciblage amphiphile a été effectué par spectrométrie de masse MALDI-TOF : spectromètre de masse couplant une source d'ionisation laser assistée par une matrice (MALDI, *Matrix-Assisted Laser Desorption/Ionisation*) et un analyseur à temps de vol (TOF, *Time-of-Flight Mass Spectrometry*). L'échantillon a d'abord été dilué au 1/50$^{ème}$ dans du tétrahydrofurane (THF), puis le mélange est dilué au 1/10$^{ème}$ dans la matrice (DHB=acide 2,5-dihydroxybenzoïque à 20 mg/mL dans THF) avant analyse par le Voyager DE STR de PerSeptive Biosystems ou équivalent.

**Exemple 1 :** Préparation des nanoparticules magnétiques (p) recouvertes d'acide oléique

1. Synthèse des nanoparticules magnétiques à base de fer

Une solution de 36 g (0,181 mole) de $FeCl_2$, $4H_2O$, 20 ml de HCl à 37% dans 150 ml de $H_2O$ a été introduite dans un mélange constitué de 3 litres d'eau et 143 ml (0,302 mole) de $FeCl_3$ à 27%. 250 ml de $NH_4OH$ à 25 % ont été introduits rapidement sous forte agitation. L'ensemble a été agité 30 mn. Les jus ont été éliminés par décantation magnétique. Le ferrofluide a été lavé 3 fois de suite avec 2 litres d'eau.

Le ferrofluide a été mis en agitation 15 mn avec 200 ml de $HNO_3$ [2M], le surnageant a été éliminé par décantation magnétique. Le ferrofluide nitrique a été porté à reflux avec 600 ml d'eau et 200 ml de $Fe(NO_3)_3$ [1M] pendant 30 mm. Le surnageant a été éliminé par décantation magnétique.

Le ferrofluide nitrique a été lavé 3 fois avec 3 litres d'acétone, puis a été repris par 400 ml d'eau. La solution a été évaporée sous vide jusqu'à un volume final de 250 ml.

| Concentration M/L | Z ave nm | Poly $\sigma$ | Diamètre XRD |
|---|---|---|---|
| 4,85 | 40 nm | 0,22 | 8,0 nm |

2. Revêtement (« coating ») des nanoparticules magnétiques à base de fer

46 ml de la solution de nanoparticules magnétiques ([Fe] = 1,14 M/l soit $7,24.10^{-4}$ M) préparée comme indiquée ci-dessus ont été dilués dans 500 ml de NaOH ($5.10^{-3}$ M). A cette solution, 60 g d'acide oléique, soit $212.10^{-3}$ M (292 équivalents molaires) ont été ajoutés. L'ensemble a été agité rapidement pendant 1h à température ambiante puis a été mis en décantation sur plaques aimantées. Le floculat a été lavé trois fois avec 500 ml d'acétone, si celui-ci n'était pas solubilisé de suite dans l'huile, et stocké en suspension dans de l'acétone.

**Exemple 2 :** Synthèse d'un ligand de ciblage amphiphile dont le ligand de ciblage est un peptidomimétique du RGD ; exemple de composé naphtyridine

Schéma de synthèse :

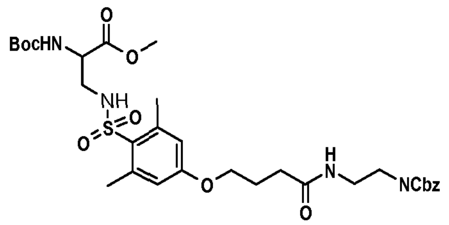

Int 1

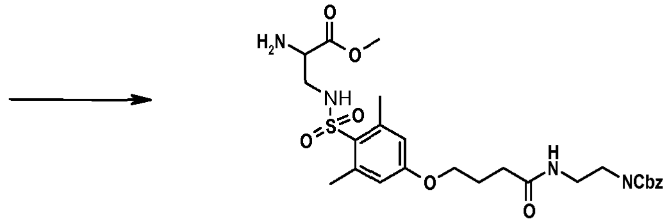

Int 2

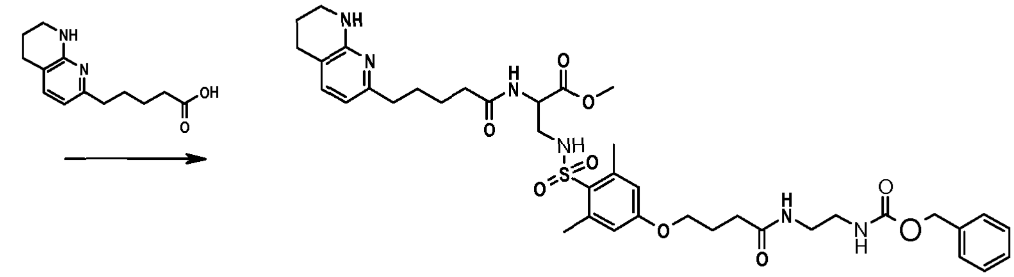

Int 3

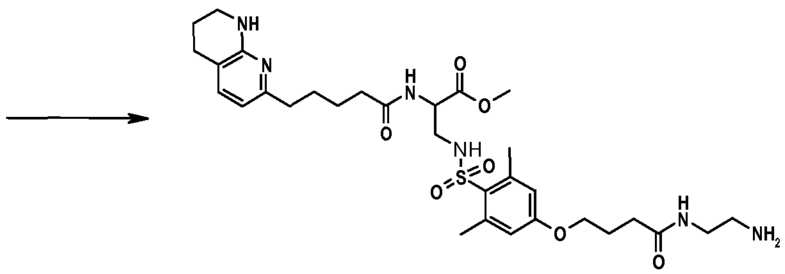

Int 4

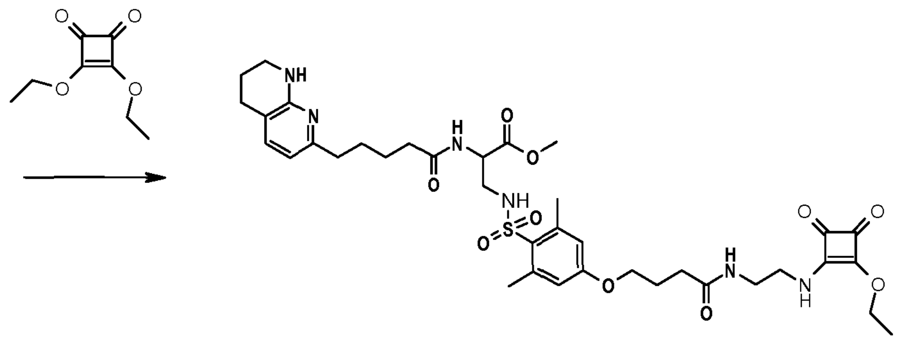

Int 5

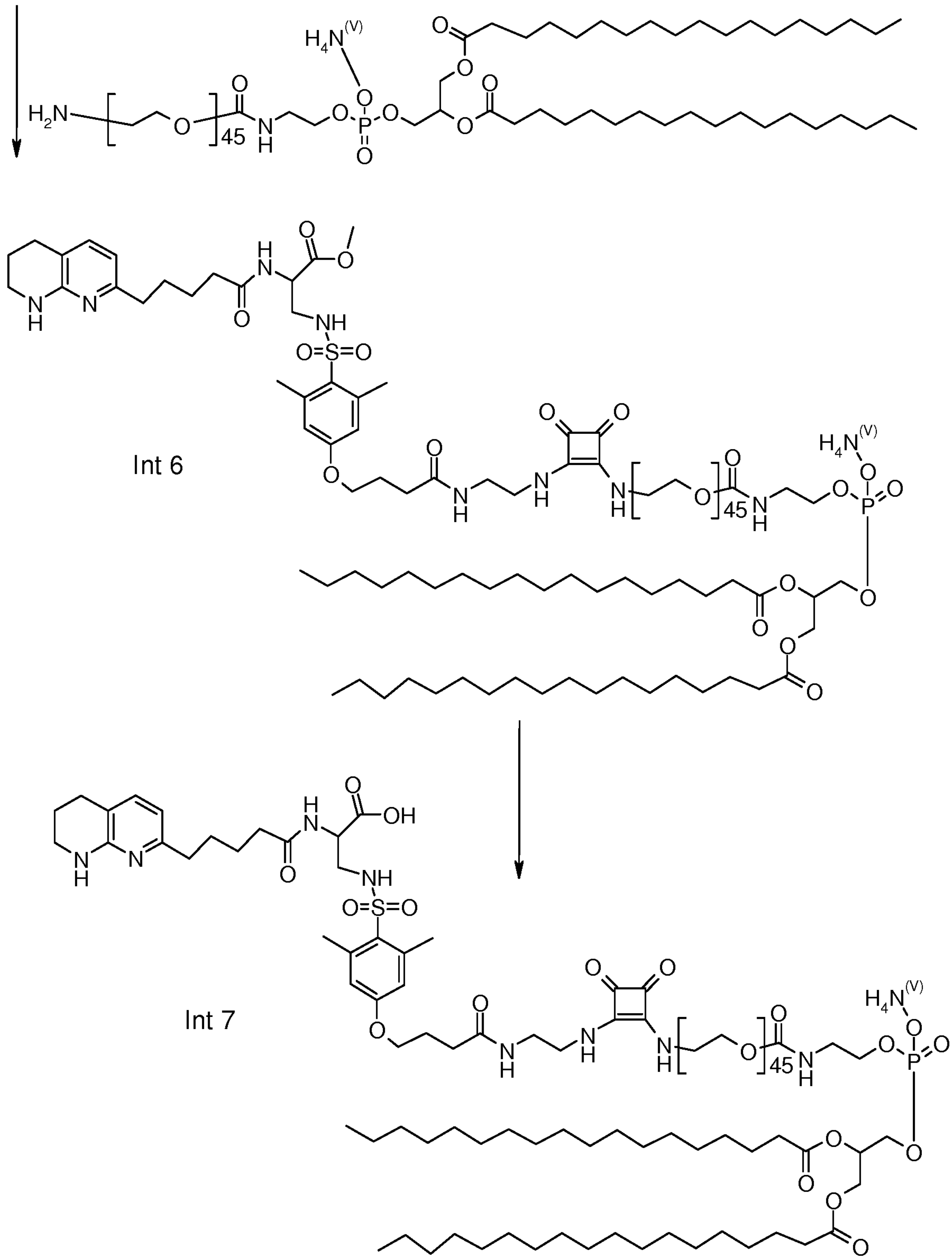

Int 6

Int 7

Etape 1 :

1g de l'int 1 ont été dissout dans 5 ml de $CH_2Cl_2$. 5ml de TFA ont été ajoutés au milieu. On a laissé 3h à TA puis évaporé à sec. On a repris dans 2x40 ml d'éther iso puis récupéré une huile que l'on a séché par évaporation. $m_{obt}$ = 0,8 g ; Rdt = 90% ; $C_{26}H_{36}N_{4O8}S$ ; MALDI-TOF: Mode positif m/z = 564

Etape 2 :

| Réactifs | Quantités | Solvants |
|---|---|---|
| Int 2 | M = 0.564g (0.001 ml) | DMF V = 10 ml |
| Acide Tetrahydronaphtyridine 2-pentanoïque | M = 0.235g (0.00023 ml) | |
| HOBT | M = 0.131g | |
| DIPEA | M = 0.286g | |
| EDCI | V = 0.2 ml | |
| Int.3 | | |

On a dissous l'acide dans le DMF puis on a introduit HOBT et EDCI et laissé 1h sous argon. On a rajouté l'int 2 et la DIPEA ; laissé 18h à TA sous argon. Après évaporation l'huile a été reprise dans $CH_2Cl_2$ et lavée par une solution diluée de $Na_2CO_3$ ; après évaporation, on obtient une huile.
$m_{obt}$ = 0,600 g; Rdt =77%; $C_{39}H_{52}N_6O_9S$ ; M/Z = 780

Etape 3:

| Réactifs | Quantités | Solvants |
|---|---|---|
| Int 3 | M = 0.6g (0.0077ml) | MeOH V = 30 ml |
| Pd/C 10 % | 1 pointe de spatule | |
| Int.4 | | |

On a dissous l'int 3 dans le méthanol et introduit la solution dans l'autoclave de 125 ml ; rajouté le catalyseur et laissé 3h sous pression d'hydrogène (P= 5 bars) à 30°C.
Après filtration du catalyseur et évaporation, on a obtenu une huile que l'on a lavée par 50 ml d'éther iso.
$m_{obt}$ = 0,300 g ; Rdt =60% ; $C_{31}H_{46}N_6O_7S$ ; HPLC = 90% ; M/Z = 646

Etape 4:

| Réactifs | Quantités | Solvants |
|---|---|---|
| Int 4 | M = 0.300g (0.000442 ml) | DMSO V = 10 ml |
| Diethyl squarate | M = 0.286g | |
| Int.5 | | TEA V = 0.25 ml |

On a dissous l'int 4 dans le DMSO puis rajouté le diethyl squarate et quelques gouttes de TEA ; Le produit est laissé 1 nuit à température ambiante sous argon puis estversé dans de l'éther : on obtient alors une pâte blanche.
$m_{obt}$ = 0,330 g ; Rdt =97%; $C_{37}H_{50}N_6O_{10}S$ ; M/Z = 770

Etape 5:

| Réactifs | Quantités | Solvants |
|---|---|---|
| Int. 5 | M = 0.330g (0.00043 ml) | |
| DSPE-$PEG_{2000}$-$NH_2$ | M = 1.07g (0.000385 ml) | |
| Solution saturée de $Na_2CO_3$ | M = 0.131g | DMSOV = 10 ml |
| Int.6 et Int 7 | | |

On a dissous l'int 5 et la DSPE-PEG2000-$NH_2$ dans le DMSO, rajouté 3 gouttes de solution saturée en $Na_2CO_3$ et 2ml d'$H_2O$. Le milieu réactionnel a été agité à température ambiante pendant 48h et a été précipité dans l'éther. La pâte obtenue a été solubilisée dans du méthanol puis elle a été purifiée sur silice, éluant $CH_2Cl_2$. Après avoir rassemblé et évaporé les fractions conformes : obtention de cristaux.

Remarque : Le produit obtenu est sous la forme acide par coupure de l'ester de méthyle par la présence de $Na_2CO_3$. On a donc obtenu directement Int 7.

$m_{obt}$ = 0,170 g ;Rdt =17% ; $C_{166}H_{308}N_9O_{63}PS$ ; M/Z = 3500

**Exemple 3 :** Synthèse d'une émulsion contenant les nanoparticules magnétiques (p) de l'exemple 1 et le ligand de ciblage amphiphile de l'exemple 2

Les nanoparticules magnétiques à base d'un composé de fer (synthétisées dans l'Exemple 1) ont été solubilisées totalement dans 60 g d'huile Miglyol® 812 à 92°C pendant 20h. La solubilisation totale desdites nanoparticules magnétiques a été appréciée visuellement en constatant l'absence d'agrégat visible à l'oeil nu. Après un retour à température ambiante, la solution a été stockée ou utilisée pour faire des émulsions.

Les tensioactifs (278,4 mg d'Egg PC (Lipoid GmbH), 55,8 mg de DSPE-PEG-2000 (Lipoid GmbH) et 27,9 mg du ligand de ciblage amphiphile de l'exemple 2) ont été dispersés à l'aide d'ultrasons dans 20 ml d'eau à 2,5 % m/m de glycérol.

5 g d'huile Miglyol® 812 comprenant les nanoparticules ont été ajoutés et pré-émulsionnés à l'« Ultra-Turrax® » à une vitesse de 25 000 tr/min (T 25).

Le volume de la pré-émulsion, avant microfluidisation, était d'environ 25 ml. La pré-émulsion a ensuite été finalisée au microfluidiseur (Microfluidics M-110-S) en recyclant pendant 3 à 4 minutes à une pression d'environ 1200 bars, ce qui correspond à environ 25 passages dans la cellule d'émulsion.

L'émulsion a été filtrée sur 0,45 $\mu$. Le volume d'émulsion récupéré était d'environ 22 ml.

**Caractéristiques de la nanoémulsion obtenue :**

| Paramètres mesurés | Résultats |
|---|---|
| Concentration en gouttelettes de nanoémulsion | 88 nM |
| Nombre de particules magnétiques par gouttelette d'huile | 150 soit environ $10^6$ Fe (une particule magnétique de maghémite de 8 nm de diamètre possède environ 10 000 molécules de Fe) |
| Concentration en Fe (mM) | 143 |
| Nombre de ligands de ciblage amphiphile (peptidomimétique RGD, composé de l'exemple 2) par gouttelette de nanoémulsion | 3500 |
| Diamètre hydrodynamique (Z ave) | 190 nm |

Cette émulsion est appelée E1 dans la suite des Exemples.

**Exemple 4 :** Mesures de relaxivité

Les mesures de relaxivité ont été réalisées sur des Relaxomètres Minispec® (Bruker Optics, Allemagne) à 60 MHz.

La solution mère a été diluée sur 5 points de gamme dans l'eau milli Q afin de pouvoir étudier la linéarité des vitesses de relaxation en fonction de la concentration. La gamme de concentrations allait de 0.12 à 1.15 mM en Fe.

La mesure de relaxivité a été effectuée à 37°C. Le dosage de Fe a été réalisé par spectroscopie d'émission

atomique sur tous les points de la gamme.

Les résultats étaient les suivants :

| Emulsion | 60MHz | |
|---|---|---|
| | r1($mM^{-1}s^{-1}$) | r2($mM^{-1}s^{-1}$) |
| E1 (selon l'invention) | 1 | 210 |

**Exemple 5 :** Détermination de la stabilité de l'émulsion

L'émulsion E1 selon l'exemple 3 a été testée en stabilité. Des mesures de taille par PCS ont été réalisées à 3, 6 et 9 mois. Les résultats sont donnés dans le tableau suivant.

| T0 | | t = 3 mois | | t = 6 mois | | t = 9 mois | |
|---|---|---|---|---|---|---|---|
| Zave (nm) | Poly σ | Zave (nm) | Poly σ | Zave (nm) | Poly σ | Zave (nm) | Poly σ |
| 193 | 0.059 | 192 | 0.043 | 191 | 0.06 | 187 | 0.064 |

**Exemple 6 :** Test de toxicité de l'émulsion E1 de l'exemple 3

**Test *in vivo***

Le test *in vivo* suivant a été effectué :

Sur Souris « swiss » d'environ 25g : Injection IV caudale vigile manuelle à 2mL/min en isovolume (200μl/animal soit 6-7 ml/kg) et suivi pendant 14 jours : mesure des paramètres hépatiques, rénaux, hématologiques à J1, J2, J7 et J14 et histologiques des organes à J14.

A 24h : anesthésie à l'isoflurane, prélèvement sublingual pour bilan hématologie sur l'automate MS4 puis exsanguination avec seringue + aiguilles héparinées.

Symptomatologie: Pas de létalité de constatée ni de signes cliniques délétères à la dose et délai testés.

Hématologie : bilan hématologique normal.

Evolution du poids des souris après infection : pas de variation pondérale significative.

Conclusion : Après analyse des résultats obtenus en hématologie ainsi que des différentes observations cliniques et post-mortem des souris, on peut conclure à l'absence d'effet toxique de la nanoémulsion selon l'invention. L'examen histologique des organes n'a pas non plus montré d'anomalie tissulaire.

**Exemple 7 :** Mesure de l'affinité (IC50) de nanoémulsions conformes à l'invention et d'autres émulsions non conformes (comparatives)

La mesure d'IC50 des émulsions a été réalisée sur des cellules HUVEC surexprimant αvβ3 par des mesures de compétitions avec de l'Echistatine $^{125}I$.

Matériels

**[0173]** - Le tampon de binding avait la composition suivante : 20 mM tris (pH 7,4), 150 mM $NaCl_2$, 1 mM $MgCl_2$, 1 mM $MnCl_2$, 0,5% BSA.

L'Echistatine (PM= 5417,12 Da) a été fournie par Bachem sous forme lyophilisée (réf H-9010 - 100μg). Elle a été reprise à 1 mg/mL en eau / TFA 0,1%.

Le précurseur iodé, 1251-SIB a une activité de 2mCi/mol. Les caractéristiques du tampon phosphate et du tampon borate étaient respectivement les suivantes : 10 mM pH 7,2 et 0.53M pH 8,5. L'albumine bovine, l'acétonitrile et l'acide trichloroacétique (TCA) étaient fournis par Sigma.

Au composé $^{125}I$-SIB (1400 μCi - 551,8MBq) coaté dans un tube en verre ont été ajoutés successivement 15 μg d'échistatine (15 μL) et le tampon borate (0,2 M final). Ce mélange réactionnel a été incubé 30 min sous agitation, à température ambiante. Le rendement de couplage a été déterminé par CCM en TCA 10%. La solution de marquage a ensuite été purifiée par gel de filtration sur colonne PD10-Séphadex G25 préalablement saturée en PBS/BSA 0,5%.

- Les caractéristiques de la solution d'échistatine-$^{125}$ISIB étaient les suivantes :

C° = 1,245μM - 6,751 μg/mL
AS = 16,02 μCi/μg

Les produits testés sont présentés dans le tableau suivant.

***Caractéristiques des produits testés***

| Produit | Concentration en gouttelettes de nanoémulsion (nM) | Concentration en Fer (mM) |
|---|---|---|
| E1T | 95 | 127 |
| E1 | 88 | 143 |
| E1' | 83 | 140 |
| E1" | 82 | 138.6 |
| E1"' | 79 | 141.3 |
| E2 | 209 | 150 |
| E3 | 115 | 129 |
| E3T | 120 | 131 |

***Gamme de concentrations testées (en moles de gouttelettes de nanoémulsion)***

| | |
|---|---|
| Echistatine | 3 μM à 0,027 nM |
| E1T | 31.67 nM à 1 aM* |
| E1 | 88 nM à 3 aM |
| E1' | 80 nM à 3 aM |
| E1" | 82 nM à 3 aM |
| E1"' | 82 nM à 3 aM |
| E2 | 69.67 nM à 1 fM |
| E3 | 115 nM à 3 aM |
| E3T | 120 nM à 3 aM |
| *aM : signifie attomolaire ($10^{-18}$ M)<br>**fM : signifie femtomolaire ($10^{-15}$ M) | |

Les émulsions E1T, E1', E1", E1"', E2, E3 et E3T ont été préparées de la même façon que l'émulsion E1 conforme à l'invention mais avec les différences suivantes :

- E1T et E3T sont dépourvues de ligand de ciblage amphiphile préparé selon le protocole de l'Exemple 2.
- E1', E1" et E1'" diffèrent principalement de l'émulsion E1, concernant leur pourcentage de ligand de ciblage amphiphile préparé selon le protocole de l'Exemple 2.
- dans E2, l'huile Miglyol® a été remplacée par de l'huile de coco.
- dans E3 et E3T, l'huile Miglyol® a été remplacée par de l'huile de soja.

L'huile de coco entre dans la définition des huiles de la phase lipidique selon l'invention (% d'Acides gras saturés en C6-C18 de 14%). L'huile de soja, par contre, ne rentre pas sous cette définition (% d'Acides gras saturés en C6-C18 de 14%).

L'huile de soja a la composition suivante

| **Nature de l'acide gras des glycérides d'acide gras** | **Concentrations (en %)** |
|---|---|
| Acide palmitique (C16:0) | 10% |
| Acide stéarique (C18:0) | 4% |
| Acide oléique (C18:1 n-9) | 23% |
| Acide linoléique (C18:2 n-6) | 51% |
| Acide alpha-Linolénique (C-18:3 n-3) | 7-10% |

- Les cellules HUVEC ont été cultivées et amplifiées en milieu EBM-2, enrichi des additifs EGM-2 (Lonza). Avant la réalisation des mesures d'affinité, les cellules HUVEC ont été traitées au PMA afin d'engendrer une surexpression de l'intégrine $\alpha v\beta 3$. A la suite de ce traitement, les cellules ont été trypsinées et une suspension cellulaire à $4.10^6$ cellules/mL en tampon binding a été réalisée.

Méthodes

La suspension des HUVEC a été distribuée dans une plaque 96 puits à fond conique, à raison de $2.10^5$ cellules dans 50 $\mu$L en tampon de binding. Cinquante $\mu$L des solutions de concentration croissante d'échistatine ou des produits ont été ajoutés par puits. Le contrôle positif a été réalisé par l'ajout de tampon de binding sans compétiteur. L'ensemble des points de concentration a été réalisé en duplicat. La plaque a été incubée 2 h à température ambiante sous agitation. Cinquante $\mu$L de la solution d'échistatine-$^{125}$I-SIB à 3 nM ont alors été distribués dans chaque puits et la plaque a de nouveau été incubée 2 h à température ambiante sous agitation. Les mélanges réactionnels ont été transférés dans des ampoules contenant 200 $\mu$L d'un coussin de densité composé de paraffine et de dibutylphtalate (10/90). Les microtubes ont ensuite été centrifugés à 12 000 rpm pendant 3 min. Les tubes ont finalement été congelés dans l'azote liquide, puis sectionnés afin de compter le culot cellulaire et le surnageant au compteur gamma. Une courbe de compétition a alors été tracée où la fixation relative de l'échistatine $^{125}$I-SIB a été déterminée par l'équation suivante :

Radioactivité fixée en présence de compétiteur (cpm)

Fixation relative de l'échistatine-I$^{125}$-SIB = Radioactivité de l'échantillon contrôle (cpm)

X 100

Les données ont été analysées à l'aide du logiciel GraphPad Prism® 5.0 qui détermine les valeurs $IC_{50}$ pour chaque produit à partir de la courbe de compétition.

Résultats

| Produit testé | % de ligand de ciblage amphiphile par rapport à la quantité totale de tensioactif (mole/mole) | IC50 (pM de gouttelettes de nanoémulsion) |
|---|---|---|
| Echistatine | - | 700 |
| Témoin E1T | 0 | 861 000 |
| E1 (selon l'invention) | 2 | 2 |
| E1' (selon l'invention) | 1 | 4 |
| E1" (selon l'invention) | 0.2 | 12 |
| E1'" (selon l'invention) | 0.05 | 91 |
| E2 (selon l'invention) | 1 | 33,18 |

(suite)

| Produit testé | % de ligand de ciblage amphiphile par rapport à la quantité totale de tensioactif (mole/mole) | IC50 (pM de gouttelettes de nanoémulsion) |
|---|---|---|
| E3 | 1 | 2278 |
| Témoin E3T | 0 | 120 000 |

On a observé une très bonne affinité pour le récepteur αvβ3 des émulsions d'USPIOs conformes à l'invention. L'affinité de l'émulsion E1 comprenant 2% (mole/mole par rapport à la quantité totale de tensioactif) de ligand de ciblage amphiphile est nettement supérieure à celle du témoin (E1T), d'environ 4 à 5 log et présente avec une relation dose/réponse dépendante de la teneur en ligand de ciblage amphiphile RGD.

Par contre, une émulsion dont la phase lipidique comprend une huile non conforme à celle de la présente invention (en l'occurrence de l'huile de soja), ne donne pas un résultat satisfaisant en termes d'affinité. Elle est trois moins affine que l'échistatine.

**Exemple 8 :** Résultats en IRM

**A- Etude de spécificité du ciblage du gliome**

1. Matériel et méthode

a) Produits testés :

- Emulsion E1 ([Fe] = 143 mM)
- Emulsion E1T ([Fe] = 127 mM

b) Culture cellulaire

Les cellules U87-MG (lignée cellulaire de glioblastome humain disponible à l'ATCC) ont été cultivées à 37°C sous atmosphère humide à 5% de CO2 et 95% d'air dans du milieu de culture DMEM low glucose supplémenté de 10% de sérum de veau néonatal inactivé et 1% de glutamine

Les cellules pour injection intracérébrale ont été obtenues par trypsination de cellules confluentes et remise en suspension dans du PBS stérile à la concentration de 1.108 cellules par mL.

c) Induction tumorale

La xénogreffe a été effectuée sur des souris nmri/nude de six semaines. La souris a été anesthésiée par injection intrapéritonéale de 10 mL/kg d'un mélange d'Imalgène 1000, de Rompun 2% et de sérum physiologique. Après anesthésie locale avec du Xylovet et désinfection à la bétadine, les cellules cancéreuses ont été injectées dans le noyau caudé selon une technique connue de l'homme du métier. L'animal a ensuite été placé en couveuse jusqu'à son réveil.

d) Innections

On a injecté les produits à tester à la dose de 100 μmol/kg.

Pour chaque souris, une carte 3D haute résolution a été réalisée post injection du produit. Cela a permis d'obtenir une carte 46 à 69 minutes.

e) Imagerie in vivo

Une IRM a été réalisée 21 jours après implantation des cellules pour repérer la présence d'une éventuelle tumeur chez les animaux induits, mesurer leur taille et ainsi sélectionner les animaux ayant une tumeur suffisamment grosse (soit un plus grand diamètre de 3 à 5 mm) pour subir une imagerie avec injection d'un des agents de contrastes attribués aux souris avant induction.

On a utilisé un imageur Bruker 2.35 Tesla. Les souris ont été anesthésiées à l'Isoflurane et maintenues à 37°C

puis placées dans une sonde IRM en quadrature (Rapid Biomed).

f) Traitement des images obtenues et analyse quantitative

Les images post-injection ont été analysées grâce à un logiciel de traitement d'image (« Post Processus Software »). Les valeurs R2* ont été comparées avec la partie saine entre les groupes d'animaux.

2. Résultats

| | E1 | E1T |
|---|---|---|
| Moyenne R2* tumeur | 22,3 | 16,2 |
| Moyenne R2* contra | 16,6 | 16,2 |
| Ecart type R2* tumeur | 2,5 | 1,92 |
| Ecart type R2* contra | 1,2 | 0,62 |

Les résultats, également présentés sous forme d'histogramme en figure 2, montrent une augmentation du R2* dans la tumeur par rapport à la partie saine lors de l'injection du produit spécifique.

D'après le test de normalité de Shapiro-Wilk, les données suivent une loi normale ce qui permet d'utiliser le test-t sur les moyennes des R2* dans la tumeur entre le produit spécifique et non-spécifique. Le résultat est significatif (p= 0,001487).

Les résultats obtenus au cours de cette étude vont dans le sens d'une spécificité de ciblage *in vivo* de l'intégrine $\alpha v \beta 3$ avec l'émulsion E1 à 2.35 Tesla et d'une différenciation possible une heure après injection du produit pour une dose de 100 μmol/kg.

L'émulsion E1 permet donc bien d'obtenir des données cliniques à partir desquelles on peut conclure à la présence d'une tumeur dans le corps d'un sujet ayant reçu ledit produit.

**B- Suivi de traitement anti-angiogénique à l'Avastin®**

1. Matériel et méthode

a) Produit utilisé

On a utilisé l'émulsion E1 (concentration en Fer = 143 mM) à la dose de 200 μmol/kg soit 1.4 mL/kg.

b) Imagerie in vivo

La même technique d'imagerie que pour l'étude de spécificité du ciblage du gliome a été utilisée.

21 jours post induction, les souris ont été sélectionnées sur le critère de taille de la tumeur. Celles pour lesquelles le plus grand axe était compris entre 3 et 5mm ont été retenues (c'est le temps J0).

Pour 13 souris (7 traitées à l'Avastin® et 6 au sérum physiologique), la chronologie était la suivante :

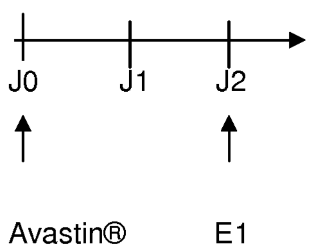


L'imagerie avec l'émulsion E1 a été réalisée 2 heures post-injection.

c) Traitement des images obtenues et analyse qualitative

Les images 2 jours post traitement et 2 jours post-injection ont été analysées grâce à un logiciel de traitement d'image (« Post Processus Software »). Une analyse qualitative des cartes R2* sur l'ensemble du volume tumoral a été réalisée. Quatre lecteurs indépendants ne connaissant pas le traitement administré, ont classé les animaux en deux groupes (« traité Avastin® » ou « traité Sérum Physiologique ») sur la base des cartes R2*. A partir des analyses des 4 lecteurs, la spécificité et la sensibilité de la méthode de suivi de traitement ont été calculées.

2. Résultats

| Lecteur | 1 | 2 | 3 | 4 | **Moyenne** |
|---|---|---|---|---|---|
| **Sensibilité (%)** | 86 | 71 | 86 | 71 | **79** |
| **Spécificité (%)** | 83 | 83 | 83 | 100 | **88** |

Le modèle expérimental a bien été validé avec une diminution de l'expression d'alpha V béta 3 chez les animaux traités à l'Avastin®.

L'efficacité de l'émulsion E1 dans le suivi d'un traitement et notamment d'un traitement anti angiogénique est ainsi démontrée. Cette émulsion E1 permet un suivi de traitement plus fiable que les autres « biomarqueurs » que sont le critère RECIST, la diffusion et le Ktrans (données de comparaison existantes mais non présentées).

## Revendications

**1.** Composition de nanoémulsion huile dans eau, comprenant :

- de 50 à 90% en poids de phase aqueuse,
- de 9,5 à 49,5% en poids de phase lipidique sous forme de nanogouttelettes comprenant :
    - une huile, et
    - des particules magnétiques (p) à base d'un composé du fer, lesdites particules magnétiques (p) étant recouvertes par un ou plusieurs acides gras en C8-C22,
- de 0,38 à 4,95% en poids d'un mélange de tensioactifs à l'interface entre les phases aqueuse et lipidique, le mélange de tensioactifs :
    - comprenant au moins un lipide amphiphile et au moins un ligand de ciblage amphiphile, et
    - représentant de 4 à 10% en poids par rapport à l'huile de la phase lipidique,

**caractérisée en ce que** l'huile de la phase lipidique comprend au moins 70% de glycérides d'acides gras saturés en C6-C18 et **en ce que** la composition comprend plus de 100 mmoles de Fer par litre de composition.

**2.** Composition selon la revendication 1, **caractérisée en ce que** le ligand de ciblage amphiphile représente 0,01 à 10% mole/mole de la quantité totale de tensioactifs.

**3.** Composition selon la revendication 1 à 2, **caractérisée en ce que** les glycérides d'acides gras saturés de l'huile de la phase lipidique sont des triglycérides d'acides gras saturés.

**4.** Composition selon l'une quelconque des revendications 1 à 3, **caractérisée en ce que** l'huile comprend des glycérides d'acides gras saturés dont les acides gras saturés sont dans les proportions suivantes:

- en C6-C18 > 70% en poids, ou
- en C6-C14 > 70% en poids ou
- en C8+C10 > 70% en poids.

5. Composition selon l'une quelconque des revendications 1 à 4, **caractérisée en ce que** l'huile comprend un mélange de diglycérides et/ou de triglycérides d'un ou plusieurs acides gras choisis parmi l'acide caprylique, l'acide caprique, l'acide linoléique, l'acide succinique et l'un des dérivés méthylés, hydroperoxylés, hydroxylés, oxoylés, époxylés, méthoxylés, halogénés, aminés, cyanylés, nitrosylés ou thiolés de l'un de ceux-ci.

6. Composition selon l'une quelconque des revendications précédentes, dans laquelle les particules magnétiques (p) comprennent ou sont constituées d'une ferrite choisie parmi la maghémite, la magnétite et un mélange de celles-ci.

7. Composition selon l'une des revendications précédentes, dans laquelle les particules magnétiques (p) sont superparamagnétiques.

8. Composition selon l'une quelconque des revendications 1 à 7, **caractérisée en ce que** le mélange de tensioactifs comprend de 80 à 96,95% mole/mole de lipide amphiphile, 3 à 15% mole/mole de lipide pégylé et 0,05 à 5% mole/mole de ligand de ciblage amphiphile.

9. Composition selon l'une quelconque des revendications 1 à 8, **caractérisée en ce que** le lipide amphiphile est un phospholipide.

10. Composition selon l'une quelconque des revendications 1 à 9, **caractérisée en ce que** le ligand de ciblage amphiphile a la formule Bio - L - Lipo, dans laquelle :

- Bio est le ligand de ciblage localisé à la surface externe des nanogouttelettes,
- Lipo est un groupe lipophile s'insérant au sein de la couche de tensioactifs
- L est un groupe de liaison reliant Bio et Lipo, avantageusement choisi parmi :

  - une simple liaison, squarate, C1-6 alkylène, PEG, par exemple $CH_2-(CH_2-O-CH_2)_k-CH_2$ avec k=1 à 10, $(CH_2)_3-NH$, $NH-(CH_2)_2-NH$, $NH-(CH_2)_3-NH$, $(CH_2)_n$, $(CH_2)_n-CO-$, $-(CH_2)_nNH-CO-$ avec n = 2 à 10 , $(CH_2CH_2O)_q(CH_2)_r-CO-$, $(CH_2CH_2O)_q(CH_2)_r-NH-CO-$avec q = 1-10 et r=2-10, $(CH_2)_n-CONH-$, $(CH_2)_n-CONH-PEG$, $(CH_2)_n-NH-$, $-OOC-CH_2-O-(CH_2)_2-O-(CH_2)_2-O-CH_2-COO-$ ; $-OOC-(CH_2)_2-CO_2-(CH_2)_2-OCO-(CH_2)_2-COO-$ ; -OOC-CH(OH)-CH(OH)-COO-; $-OOC-(CH_2)_n-COO-$; $-NH-(CH_2)_n-NH-$, avec n=0-20; $NH-(CH_2)_n-CO_2$ ; $NH-CH_2-(CH_2-O-CH_2)_n-CO_2$ avec n=1 à 10,
  - P1-I-P2, P1 et P2 étant indépendamment choisis parmi O, S, NH, une liaison simple, $CO_2$, NHCO, CONH, NHCONH, NHCSNH, $SO_2NH$- $NHSO_2$- squarate avec I = alkylène, alkoxyalkylène, polyalkoxyalkylène (notamment PEG), alkylène interrompu par un ou plusieurs squarates, par un ou plusieurs aryles, avantageusement phényles, ou par un ou plusieurs groupes choisis parmi -NH-, -O-, -CO-, -NH(CO)-, -(CO)NH-, -O(CO)-, et -(OC)O-, alkenylène ou alkynylène, lesdits alkenylène ou alkynylène étant éventuellement interrompus par un ou plusieurs groupes choisis parmi -NH-, -O-, -CO-, -NH(CO)-, -(CO)NH-, -O(CO)-, ou -(OC)O-.

11. Composition selon l'une quelconque des revendications 1 à 10, **caractérisée en ce que** le ligand de ciblage du ligand de ciblage amphiphile est choisi parmi : les peptides, les pseudopeptides, les peptidomimétiques, les acides aminés, les agents de ciblage d'intégrines, les glycoprotéines, les lectines, les dérivés ptéroïques ou aminoptéroïques, les dérivés de l'acide folique et antifolique, les anticorps ou fragments d'anticorps, les stéroïdes, les oligonucléotides, les séquences d'acide ribonucléique, les séquences d'acide désoxyribonucléique, les hormones, les protéines éventuellement recombinantes ou mutées, les mono ou polysaccharides, les composés à squelette benzothiazole, benzofurane, styrylbenzoxazole/thiazole/imidazole / quinoline, styrylpiridine.

12. Composition selon l'une quelconque des revendications 1 à 11, **caractérisée en ce que** le ligand de ciblage du ligand de ciblage amphiphile est un ligand d'une cible associée à l'angiogenèse.

13. Composition selon l'une quelconque des revendications 1 à 12, **caractérisée en ce que** le ligand de ciblage amphiphile a la formule :

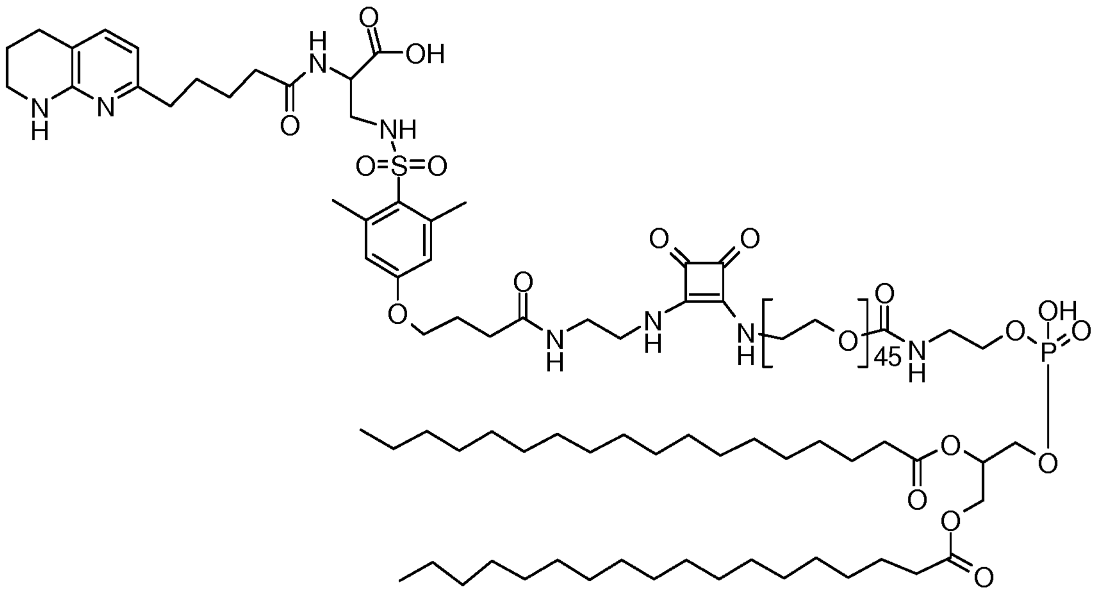

ou un de ses sels.

**14.** Procédé de préparation d'une composition selon les revendications 1 à 13, comprenant les étapes de :

a) Solubilisation de particules magnétiques (p) à base d'un composé de fer recouvertes par un ou plusieurs acides gras en C8-C22, dans une huile comprenant au moins 70% en poids de glycérides d'acides gras saturés en C6-C18 pour former une phase lipidique,
b) Mélange de la phase lipidique obtenue à l'étape a) et d'une phase aqueuse comprenant le mélange de tensioactifs, de manière à former des nanogoutelettes,
c) récupération de la nanoémulsion obtenue.

**15.** Produit de contraste comprenant la composition de nanoémulsion selon l'une quelconque des revendications 1 à 13.

**16.** Produit de contraste comprenant la composition de nanoémulsion selon la revendication 12 ou 13.

**17.** Méthode d'imagerie du corps entier ou d'une partie du corps d'un individu comprenant une étape d'obtention d'une ou plusieurs images du corps entier ou d'une partie du corps d'un individu par une technique d'imagerie médicale, dans laquelle ledit corps entier ou ladite partie du corps de l'individu comprend la composition selon l'une quelconque des revendications 1 à 13 ou le produit de contraste selon la revendication 15 ou 16 et dans laquelle la ou les image(s) sont associée(s) aux particules magnétiques à base d'un composé du fer contenues dans la composition selon l'une quelconque des revendications 1 à 13 ou dans le produit de contraste selon la revendication 15 ou 16.

**18.** Composition selon la revendication 12 ou 13 ou produit de contraste selon la revendication 16 destiné(e) à être utilisée dans une méthode d'évaluation « in vivo » de l'angiogenèse chez un individu comprenant les étapes consistant à :

a) obtenir une ou plusieurs images du corps entier ou d'une partie du corps d'un individu par une technique d'imagerie médicale, dans laquelle ledit corps entier ou ladite partie du corps de l'individu comprend la composition selon la revendication 12 ou 13 ou le produit de contraste selon la revendication 16 et dans laquelle la ou les image(s) est(sont) associée(s) aux particules magnétiques à base d'un composé du fer contenues dans la composition selon la revendication 12 ou 13 ou dans le produit de contraste selon la revendication 16 ;
b) évaluer l'angiogenèse dans le corps entier ou la partie du corps de l'individu à partir de la ou des images obtenues à l'étape a).

**19.** Composition selon la revendication 12 ou 13 ou produit de contraste selon la revendication 16 pour son utilisation dans une méthode d'évaluation de l'efficacité d'un traitement anti-angiogénique comprenant les étapes consistant à :

a) évaluer l'angiogénèse chez un individu ayant reçu un traitement anti-angiogénique, par la méthode telle que

définie à la revendication 18 ;
b) comparer l'angiogenèse évaluée selon l'étape a) avec une référence de l'angiogénèse chez l'individu avant le traitement anti-angiogénique,

ladite méthode n'incluant pas d'étape d'administration du traitement anti-angiogénique.

**20.** Composition selon la revendication 12 ou 13 ou produit de contraste selon la revendication 16 pour son utilisation dans une méthode d'évaluation de l'efficacité d'un traitement anti-angiogénique comprenant les étapes consistant à :

c) évaluer l'angiogénèse chez un individu ayant reçu un traitement anti-angiogénique, par la méthode selon la revendication 18 ;
d) comparer l'angiogenèse évaluée selon l'étape a) avec une référence de l'angiogénèse chez l'individu avant le traitement anti-angiogénique.

## Patentansprüche

**1.** Zusammensetzung einer Öl-in-Wasser-Nanoemulsion, umfassend:

- 50 bis 90 Gew.-% wässrige Phase,
- 9,5 bis 49,5 Gew.-% Lipidphase in Form von Nanotröpfchen, umfassend:

  - ein Öl und
  - Magnetteilchen (p) auf Basis einer Eisenverbindung, wobei die Magnetteilchen (p) mit einer oder mehreren C8-C22-Fettsäuren umhüllt sind,

- 0,38 bis 4,95 Gew.-% eines Tensidgemischs an der Grenzfläche zwischen der wässrigen Phase und der Lipidphase, wobei das Tensidgemisch:

  - mindestens ein amphiphiles Lipid und mindestens einen amphiphilen Targeting-Liganden umfasst, und
  - 4 bis 10 Gew.-% bezogen auf das Öl der Lipidphase ausmacht,

**dadurch gekennzeichnet, dass** das Öl der Lipidphase mindestens 70 % Glyceride von gesättigten C6-C18-Fettsäuren umfasst und dadurch, dass die Zusammensetzung mehr als 100 mmol Eisen pro Liter Zusammensetzung umfasst.

**2.** Zusammensetzung nach Anspruch 1, **dadurch gekennzeichnet, dass** der amphiphile Targeting-Ligand 0,01 bis 10% mol/mol der Tensid-Gesamtmenge ausmacht.

**3.** Zusammensetzung nach Anspruch 1 bis 2, **dadurch gekennzeichnet, dass** die Glyceride der gesättigten Fettsäuren des Öls der Lipidphase Triglyceride von gesättigten Fettsäuren sind.

**4.** Zusammensetzung nach einem der Ansprüche 1 bis 3, **dadurch gekennzeichnet, dass** das Öl Glyceride von gesättigten Fettsäuren umfasst, deren gesättigte Fettsäuren in den folgenden Anteilen vorliegen:

- C6-C18 > 70 Gew.-% oder
- C6-C14 > 70 Gew.-% oder
- C8+C10 > 70 Gew.-%.

**5.** Zusammensetzung nach einem der Ansprüche 1 bis 4, **dadurch gekennzeichnet, dass** das Öl ein Gemisch aus Diglyceriden und/oder Triglyceriden einer oder mehrerer Fettsäuren umfasst, ausgewählt aus Caprylsäure, Caprinsäure, Linolsäure, Bernsteinsäure und einem der Methyl-, Hydroperoxyl-, Hydroxyl-, Oxoyl-, Epoxyl-, Methoxyl-, Halogen-, Amin-, Cyanyl-, Nitrosyl- oder Thiolderivate von einer davon.

**6.** Zusammensetzung nach einem der vorhergehenden Ansprüche, wobei die Magnetteilchen (p) ein Ferrit, ausgewählt aus Maghemit, Magnetit und einem Gemisch dieser beiden, umfassen oder daraus bestehen.

**7.** Zusammensetzung nach einem der vorhergehenden Ansprüche, wobei die Magnetteilchen (p) superparamagnetisch

sind.

**8.** Zusammensetzung nach einem der Ansprüche 1 bis 7, **dadurch gekennzeichnet, dass** das Tensidgemisch 80 bis 96,95 % mol/mol amphiphiles Lipid, 3 bis 15 % mol/mol pegyliertes Lipid und 0,05 bis 5% mol/mol amphiphilen Targeting-Liganden umfasst.

**9.** Zusammensetzung nach einem der Ansprüche 1 bis 8, **dadurch gekennzeichnet, dass** das amphiphile Lipid ein Phospholipid ist.

**10.** Zusammensetzung nach einem der Ansprüche 1 bis 9, **dadurch gekennzeichnet, dass** der amphiphile Targeting-Ligand die Formel Bio - L - Lipo aufweist, wobei:

- Bio der Targeting-Ligand ist, der auf der äußeren Oberfläche der Nanotröpfchen liegt,
- Lipo eine lipophile Gruppe ist, die sich in die Tensidschicht einfügt;
- L eine Bindungsgruppe ist, die Bio und Lipo verbindet, vorteilhafterweise ausgewählt aus:

    ▪ einer einfachen Bindung, Squarat, C1-6-Alkylen, PEG, zum Beispiel $CH_2$-($CH_2$-O-$CH_2$)$_k$-$CH_2$ mit k=1 bis 10, ($CH_2$)$_3$-NH, NH-($CH_2$)$_2$-NH, NH-($CH_2$)$_3$-NH, ($CH_2$)$_n$, ($CH_2$)$_n$-CO-, - ($CH_2$)$_n$NH-CO- mit n = 2 bis 10, ($CH_2CH_2O$)$_q$($CH_2$)$_r$-CO-, ($CH_2CH_2O$)$_q$($CH_2$)$_r$-NH-CO- mit q = 1-10 und r = 2-10, ($CH_2$)$_n$-CONH-, ($CH_2$)$_n$-CONH-PEG, ($CH_2$)$_n$-NH-, -OOC-$CH_2$-O-($CH_2$)$_2$-O-($CH_2$)$_2$-O-$CH_2$-COO-; -OOC-($CH_2$)$_2$-$CO_2$-($CH_2$)$_2$-OCO-($CH_2$)$_2$-COO-; -OOC-CH(OH)-CH(OH)-COO-; -OOC-($CH_2$)$_n$-COO-; -NH-($CH_2$),-NH-, mit n= 0-20; NH-($CH_2$)$_n$-$CO_2$; NH-$CH_2$-($CH_2$-O-$CH_2$)$_n$-$CO_2$ mit n = 1 bis 10,

    ▪ P1-I-P2, P1 und P2 unabhängig ausgewählt sind aus O, S, NH, einer einfachen Bindung, $CO_2$, NHCO, CONH, NHCONH, NHCSNH, $SO_2$NH-, NH$SO_2$-, Squarat mit I = Alkylen, Alkoxyalkylen, Polyalkoxyalkylen (insbesondere PEG), Alkylen, das von einem oder mehreren Squaraten, von einem oder mehreren Arylen, vorteilhafterweise Phenylen, oder von einer oder mehreren Gruppen, ausgewählt aus -NH-, -O-, -CO-, -NH(CO)-, -(CO)NH-, - O(CO)- und -(OC)O-, Alkenylen oder Alkinylen unterbrochen ist, wobei das Alkenylen oder das Alkinylen gegebenenfalls von einer oder mehreren Gruppen, ausgewählt aus -NH-, - O-, -CO-, -NH(CO)-, -(CO)NH-, -O(CO)- oder -(OC)O-unterbrochen ist.

**11.** Zusammensetzung nach einem der Ansprüche 1 bis 10, **dadurch gekennzeichnet, dass** der Targeting-Ligand des amphiphilen Targeting-Liganden ausgewählt ist aus: Peptiden, Pseudopeptiden, Peptidomimetika, Aminosäuren, Mittel zum Integrin-Targeting, Glycoproteinen, Lectinen, Pteroin- oder Aminopteroinderivaten, Fol- und Antifolsäurederivaten, Antikörpern oder Antikörperfragmenten, Steroiden, Oligonukleotiden, Ribonukleinsäuresequenzen, Desoxyribonukleinsäuresequenzen, Hormonen, gegebenenfalls rekombinanten oder mutierten Proteinen, Mono- oder Polysacchariden, Verbindungen mit Benzothiazol-, Benzofuran-, Styrylbenzoxazol-/Thiazol-/Imidazol-/Chinolin-, Styrylpiridin-Hauptkette.

**12.** Zusammensetzung nach einem der Ansprüche 1 bis 11, **dadurch gekennzeichnet, dass** der Targeting-Ligand des amphiphilen Targeting-Liganden ein Ligand eines Targets ist, das mit der Angiogenese assoziiert ist.

**13.** Zusammensetzung nach einem der Ansprüche 1 bis 12, **dadurch gekennzeichnet, dass** der amphiphile Targeting-Ligand die folgende Formel aufweist:

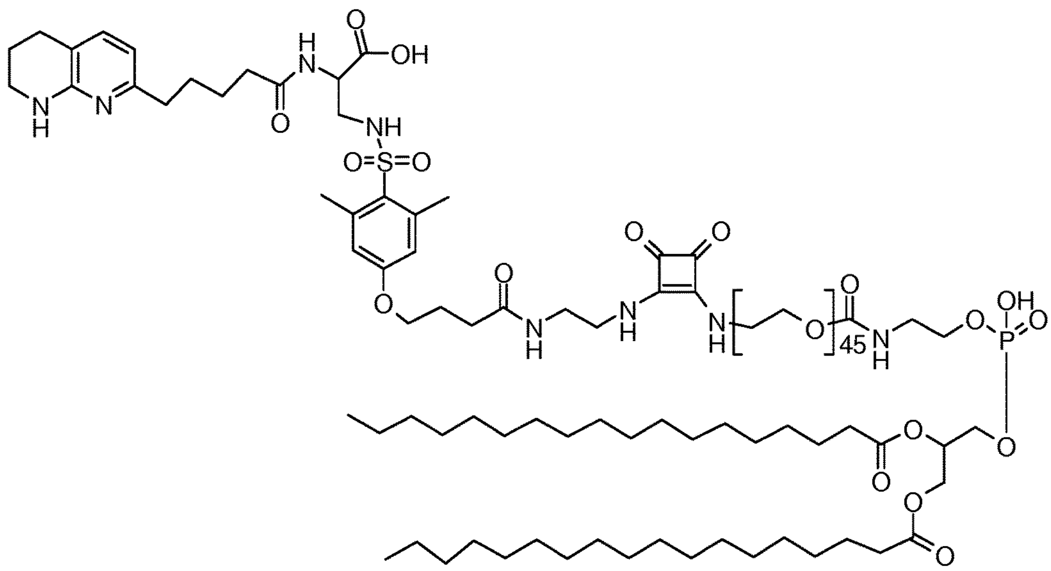

oder eines seiner Salze.

**14.** Verfahren zur Herstellung einer Zusammensetzung nach den Ansprüchen 1 bis 13, das die folgenden Schritte umfasst:

a) Solubilisieren von Magnetteilchen (p), die auf einer Eisenverbindung basieren, die mit einer oder mehreren C8-C22 Fettsäuren umhüllt sind, in einem Öl, umfassend mindestens 70 Gew.-% Glyceride von gesättigten C6-C18-Fettsäuren, um eine Lipidphase zu bilden,
b) Mischen der in Schritt a) erhaltenen Lipidphase und einer wässrigen Phase, umfassend das Tensidgemisch, um Nanotröpfchen zu bilden,
c) Gewinnen der erhaltenen Nanoemulsion.

**15.** Kontrastprodukt, umfassend die Nanoemulsion-Zusammensetzung nach einem der Ansprüche 1 bis 13.

**16.** Kontrastprodukt, umfassend die Nanoemulsion-Zusammensetzung nach Anspruch 12 oder 13.

**17.** Bildgebendes Verfahren für den ganzen Körper oder einen Teil des Körpers eines Individuums, umfassend einen Schritt zum Erhalten eines oder mehrerer Bilder des ganzen Körpers oder eines Teils des Körpers eines Individuums mit einer medizinischen Bildgebungstechnik, wobei der ganze Körper oder der Teil des Körpers des Individuums die Zusammensetzung nach einem der Ansprüche 1 bis 13 oder das Kontrastprodukt nach Anspruch 15 oder 16 umfasst und wobei das oder die Bilder mit den Magnetteilchen auf Basis einer Eisenverbindung assoziiert ist (sind), die in der Zusammensetzung nach einem der Ansprüche 1 bis 13 oder in dem Kontrastprodukt nach Anspruch 15 oder 16 enthalten sind.

**18.** Zusammensetzung nach Anspruch 12 oder 13 oder Kontrastprodukt nach Anspruch 16, die (das) dazu bestimmt ist, in einem Verfahren zur "In-vivo"-Evaluierung der Angiogenese bei einem Individuum verwendet zu werden, das die folgenden Schritte umfasst:

a) Erhalten eines oder mehrerer Bilder des ganzen Körpers oder eines Teils des Körpers eines Individuums mit einer medizinischen Bildgebungstechnik, wobei der ganze Körper oder der Teil des Körpers des Individuums die Zusammensetzung nach Anspruch 12 oder 13 oder das Kontrastprodukt nach Anspruch 16 umfasst, und wobei das oder die Bild(er) mit den Magnetteilchen auf Basis einer Eisenverbindung, die in der Zusammensetzung nach Anspruch 12 oder 13 oder in dem Kontrastprodukt nach Anspruch 16 enthalten sind, assoziiert ist (sind);
b) Evaluieren der Angiogenese in dem ganzen Körper oder dem Teil des Körpers des Individuums ausgehend von dem oder den in Schritt a) erhaltenen Bildern.

**19.** Zusammensetzung nach Anspruch 12 oder 13 oder Kontrastprodukt nach Anspruch 16 zu deren (dessen) Verwendung in einem Verfahren zur Evaluierung der Wirksamkeit einer Antiangiogenesebehandlung, das die folgenden Schritte umfasst:

a) Evaluieren der Angiogenese in einem Individuum, das eine Antiangiogenesebehandlung erhalten hat, mit dem Verfahren wie in Anspruch 18 definiert;
b) Vergleichen der evaluierten Angiogenese gemäß Schritt a) mit einer Referenz der Angiogenese bei dem Individuum vor der Antiangiogenesebehandlung,

wobei das Verfahren keinen Schritt zur Verabreichung der Antiangiogenesebehandlung beinhaltet.

**20.** Zusammensetzung nach Anspruch 12 oder 13 oder Kontrastprodukt nach Anspruch 16 zu deren (dessen) Verwendung in einem Verfahren zur Evaluierung der Wirksamkeit einer Antiangiogenesebehandlung, das die folgenden Schritte umfasst:

c) Evaluieren der Angiogenese in einem Individuum, das eine Antiangiogenesebehandlung erhalten hat, mit dem Verfahren nach Anspruch 18;
d) Vergleichen der evaluierten Angiogenese gemäß Schritt a) mit einer Referenz der Angiogenese bei dem Individuum vor der Antiangiogenesebehandlung.

## Claims

**1.** Oil-in-water nanoemulsion composition comprising:

- from 50% to 90% by weight of aqueous phase,
- from 9.5% to 49.5% by weight of lipid phase in the form of nanodroplets comprising:
  - an oil and
  - magnetic particles (p) based on an iron compound, said magnetic particles (p) being covered with one or more C8-C22 fatty acids,
- from 0.38% to 4.95% by weight of a mixture of surfactants at the interface between the aqueous and lipid phases, the mixture of surfactants:
  - comprising at least one amphiphilic lipid and at least one amphiphilic targeting ligand, and
  - representing from 4% to 10% by weight relative to the oil of the lipid phase,

**characterized in that** the oil of the lipid phase comprises at least 70% of glycerides of C6-C18 saturated fatty acids and **in that** the composition comprises more than 100 mmol of iron per liter of composition.

**2.** Composition according to Claim 1, **characterized in that** the amphiphilic targeting ligand represents 0.01% to 10% mol/mol of the total amount of surfactants.

**3.** Composition according to Claim 1 or 2, **characterized in that** the glycerides of saturated fatty acids in the oil of the lipid phase are triglycerides of saturated fatty acids.

**4.** Composition according to any one of Claims 1 to 3, **characterized in that** the oil comprises glycerides of saturated fatty acids where the proportions of the saturated fatty acids are as follows:

- C6-C18 > 70% by weight, or
- C6-C14 > 70% by weight, or
- C8+C10 > 70% by weight.

**5.** Composition according to any one of Claims 1 to 4, **characterized in that** the oil comprises a mixture of diglycerides and/or triglycerides of one or more fatty acids selected from caprylic acid, capric acid, linoleic acid, succinic acid, and one of the methyl, hydroperoxyl, hydroxyl, oxoyl, epoxy, methoxy, halo, amino, cyano, nitrosyl, or thiol derivatives of any of said acids.

**6.** Composition according to any one of the preceding claims, wherein the magnetic particles (p) comprise or are composed of a ferrite selected from maghemite, magnetite, and a mixture thereof.

**7.** Composition according to one of the preceding claims, wherein the magnetic particles (p) are superparamagnetic.

**8.** Composition according to any one of Claims 1 to 7, **characterized in that** the mixture of surfactants comprises from 80% to 96.95% mol/mol of amphiphilic lipid, 3% to 15% mol/mol of pegylated lipid, and 0.05% to 5% mol/mol of amphiphilic targeting ligand.

**9.** Composition according to any one of Claims 1 to 8, **characterized in that** the amphiphilic lipid is a phospholipid.

**10.** Composition according to any one of Claims 1 to 9, **characterized in that** the amphiphilic targeting ligand has the formula Bio - L - Lipo, in which:

- Bio is the targeting ligand located on the outer surface of the nanodroplets,
- Lipo is a lipophilic group for insertion within the layer of surfactants,
- L is a linking group connecting Bio and Lipo, advantageously selected from:

  - a single bond, squarate, C1-6 alkylene, PEG, for example $CH_2-(CH_2-O-CH_2)_k-CH_2$ with k = 1 to 10, $(CH_2)_3-NH$, $NH-(CH_2)_2-NH$, $NH-(CH_2)_3-NH$, $(CH_2)_n$, $(CH_2)_n-CO-$, $-(CH_2)_nNH-CO-$ with n = 2 to 10, $(CH_2CH_2O)_q(CH_2)_r-CO-$, $(CH_2CH_2O)_q(CH_2)_r-NH-CO-$ with q = 1-10 and r = 2-10, $(CH_2)_n-CONH-$, $(CH_2)_n-CONH-PEG$, $(CH_2)_n-NH-$, $-OOC-CH_2-O-(CH_2)_2-O-(CH_2)_2-O-CH_2-COO-$; $-OOC-(CH_2)_2-CO_2-(CH_2)_2-OCO-(CH_2)_2-COO-$; $-OOC-CH(OH)-CH(OH)-COO-$; $-OOC-(CH_2)_n-COO-$; $-NH-(CH_2)_n-NH-$, with n = 0-20; $NH-(CH_2)_n-CO_2$; $NH-CH_2-(CH_2-O-CH_2)_n-CO_2$ with n = 1 to 10,
  - P1-I-P2, where P1 and P2 are selected independently from O, S, NH, a single bond, $CO_2$, NHCO, CONH, NHCONH, NHCSNH, $SO_2NH-$, $NHSO_2-$, and squarate where I = alkylene, alkoxyalkylene, polyalkoxyalkylene (especially PEG), alkylene interrupted by one or more squarates, by one or more aryls, advantageously phenyls, or by one or more groups selected from -NH-, -O-, -CO-, -NH(CO)-, -(CO)NH-, -O(CO)-, and -(OC)O-, alkenylene or alkynylene, said alkenylene or alkynylene being optionally interrupted by one or more groups selected from -NH-, -O-, -CO-, -NH(CO)-, -(CO)NH-, - O(CO)-, or -(OC)O-.

**11.** Composition according to any one of Claims 1 to 10, **characterized in that** the targeting ligand of the amphiphilic targeting ligand is selected from the following: peptides, pseudopeptides, peptidomimetics, amino acids, integrin targeting agents, glycoproteins, lectins, pteroic or aminopteroic derivatives, folic and antifolic acid derivatives, antibodies or antibody fragments, steroids, oligonucleotides, ribonucleic acid sequences, deoxyribonucleic acid sequences, hormones, proteins, which are optionally recombinant or mutated, mono- or polysaccharides, compounds having a benzothiazole, benzofuran, styrylbenzoxazole/thiazole/imidazole/quinoline, or styrylpyridine structure.

**12.** Composition according to any one of Claims 1 to 11, **characterized in that** the targeting ligand of the amphiphilic targeting ligand is a ligand of a target associated with angiogenesis.

**13.** Composition according to any one of Claims 1 to 12, **characterized in that** the amphiphilic targeting ligand has the formula:

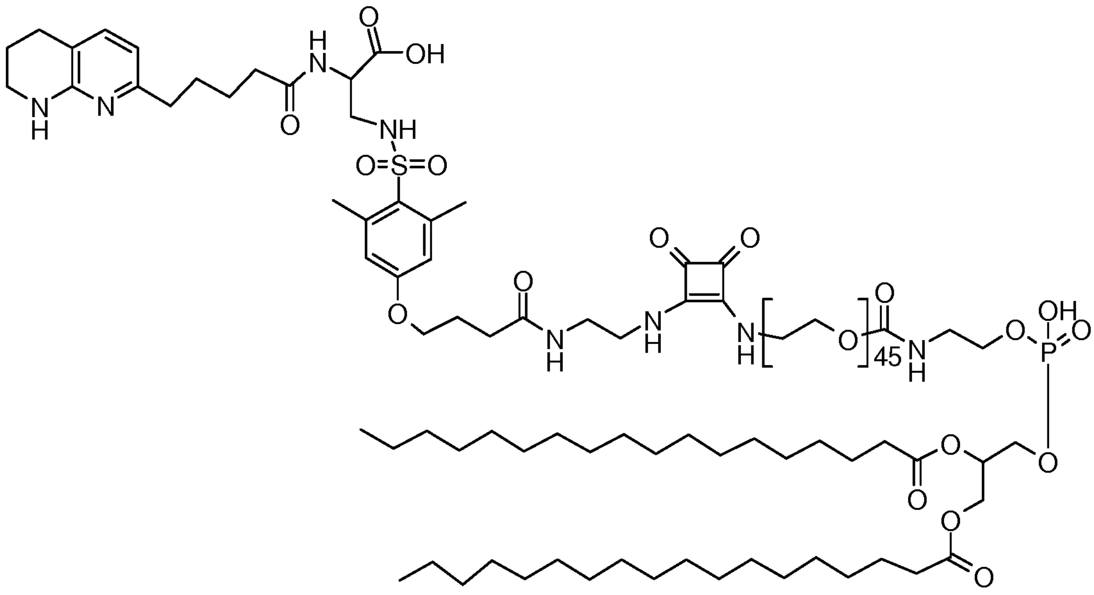

or a salt thereof.

**14.** Process for preparing a composition according to Claims 1 to 13, comprising the steps of:

a) dissolving magnetic particles (p) based on an iron compound and covered with one or more C8-C22 fatty acids in an oil comprising at least 70% by weight of glycerides of C6-C18 saturated fatty acids, to form a lipid phase,
b) mixing the lipid phase obtained in step a) with an aqueous phase comprising the mixture of surfactants, so as to form nanodroplets,
c) recovering the resulting nanoemulsion.

**15.** Contrast product comprising the nanoemulsion composition according to any one of Claims 1 to 13.

**16.** Contrast product comprising the nanoemulsion composition according to Claim 12 or 13.

**17.** Method for imaging the whole body or a part of the body of an individual, comprising a step of obtaining one or more images of the whole body or of a part of the body of an individual by a medical imaging technique, wherein said whole body or said part of the body of the individual comprises the composition according to any one of claims 1 to 13 or the contrast product according to claim 15 or 16, and wherein the one or more images are associated with the magnetic particles based on an iron compound that are contained in the composition according to any one of Claims 1 to 13 or in the contrast product according to Claim 15 or 16.

**18.** Composition according to Claim 12 or 13 or contrast product according to Claim 16 for use thereof in a method for evaluating angiogenesis "in vivo" in an individual, comprising the steps of:

a) obtaining one or more images of the whole body or of a part of the body of an individual by a medical imaging technique, wherein said whole body or said part of the body of the individual comprises the composition according to Claim 12 or 13 or the contrast product according to Claim 16 and wherein the one or more images are associated with the magnetic particles based on an iron compound that are contained in the composition according to Claim 12 or 13 or in the contrast product according to Claim 16;
b) evaluating the angiogenesis in the whole body or the part of the body of the individual on the basis of the one or more images obtained in step a).

**19.** Composition according to Claim 12 or 13 or contrast product according to Claim 16 for use thereof in a method for evaluating the efficacy of an antiangiogenic treatment, comprising the steps of:

a) evaluating angiogenesis in an individual having received an antiangiogenic treatment, by the method as defined in Claim 18;

b) comparing the angiogenesis evaluated according to step a) with an angiogenesis reference in the individual before the antiangiogenic treatment,

said method not including a step of administering the antiangiogenic treatment.

**20.** Composition according to Claim 12 or 13 or contrast product according to Claim 16 for use thereof in a method for evaluating the efficacy of an antiangiogenic treatment, comprising the steps of:

c) evaluating angiogenesis in an individual having received an antiangiogenic treatment, by the method according to Claim 18;
d) comparing the angiogenesis evaluated according to step a) with an angiogenesis reference in the individual before the antiangiogenic treatment.

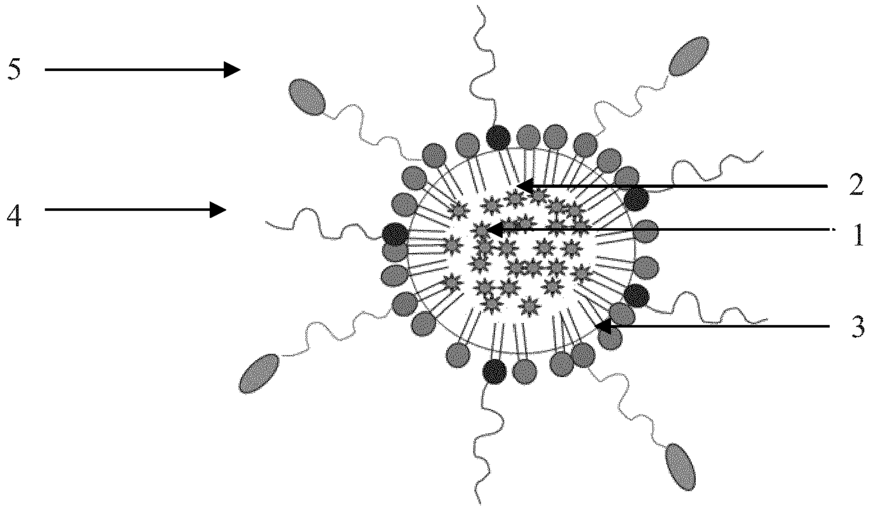


Figure 1

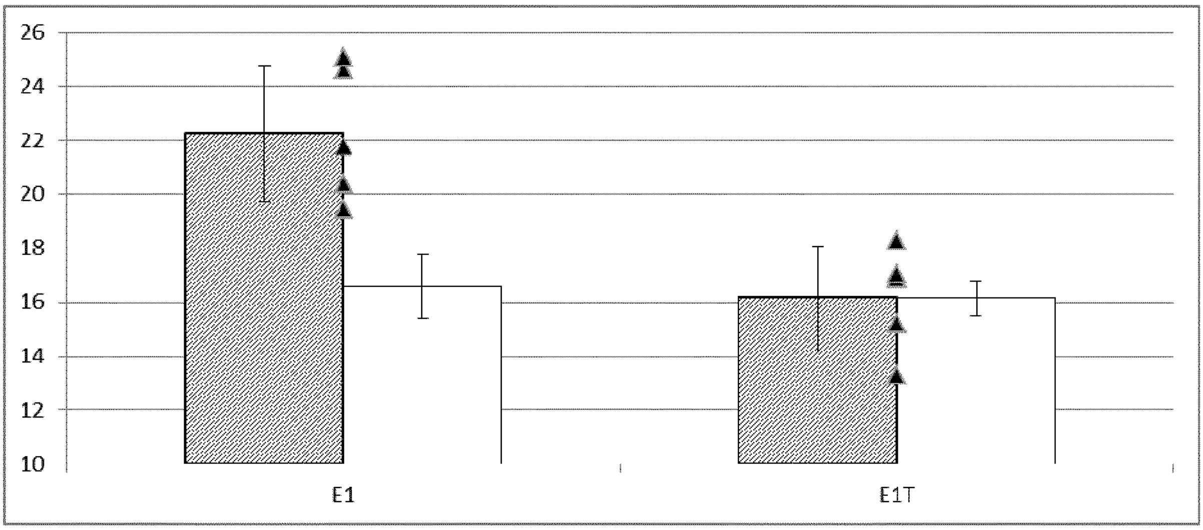


Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005014051 A
- US 20100297019 A
- WO 2004112839 A
- WO 2007042504 A
- US 20050227985 A
- WO 2010102238 A
- WO 0197850 A
- US 6372194 B
- WO 0109188 A
- WO 0177145 A
- WO 0226776 A
- WO 02081497 A
- WO 03062198 A
- WO 0160416 A
- WO 9940947 A
- WO 02062810 A
- WO 0240060 A
- US 6524554 B
- WO 02094873 A
- US 6489333 B
- US 6511648 B
- US 20020106325 A
- WO 0197861 A
- WO 0198294 A
- WO 0110450 A
- US 6261535 B
- US 5707605 A
- WO 0228441 A
- WO 02056670 A
- US 6410695 B
- US 6391280 B
- WO 9954317 A
- US 6491893 B
- US 20020128553 A
- WO 02054088 A
- WO 0232292 A
- WO 0238546 A
- WO 03006059 A
- US 6534038 B
- WO 0177102 A
- EP 1121377 A
- WO 0244156 A
- WO 02059110 A
- WO 9400489 A
- WO 9405269 A
- WO 02066512 A
- EP 0425212 A
- US 6537520 B
- WO 2003014145 A
- WO 0320701 A
- WO 0311115 A
- WO 02085903 A
- US 2007098631 A
- WO 2009114776 A
- WO 2007042506 A
- US 6010682 A


### Littérature non-brevet citée dans la description


- **MANDAL et al.** *Langmuir,* 2005, vol. 21 (9
- **JARZYNA et al.** *Biomaterials,* 2009, vol. 30, 6947-6954
- **SENPAN et al.** *JACS,* 2009, vol. 3 (12), 3917-3926
- **J. LYKLEMA et al.** *Materials Science Research,* 1984, vol. 17, 1-24
- **MÜLLER et al.** *Eur. J. Org. Chem,* 2002, 3966-3973
- *Pharmacological Reviews,* vol. 52 (2), 179
- **W.KRAUSE.** Topics in Current Chemistry. Springer, vol. 222
- *Bioorganic & Medicinal Chemistry,* 2003, vol. 11, 1319-1341
- *Nuclear Medicine Communications,* 1999, vol. 20
- *Topics in Current Chemistry,* vol. 222, 260-274
- *J Med. Chem.,* 2003, vol. 46, 4790-4798
- *Bioorg. Med. Chem. Letters,* 2004, vol. 14, 4515-4518
- *Bioorg. Med. Chem. Letters,* 2005, vol. 15, 1647-1650
- **R. PECORA.** *J. of Nano. Res.,* 2000, vol. 2, 123-131